# EUROPÄISCHE PATENTSCHRIFT

(11) **EP 3 886 699 B1**
(45) Veröffentlichungstag und Bekanntmachung des Hinweises auf die Patenterteilung: **20.08.2025**
(21) Anmeldenummer: 19809408.8
(22) Anmeldetag: 20.11.2019
(51) Int. Cl.: A61B 5/097, G01N 21/77, G01N 21/03, G01N 33/497, G01N 21/64, G01N 21/05, B29C 45/14, B29C 45/16

(54) **VON ATEMGAS DURCHSTRÖMBARE MESSVORRICHTUNG ZUR MESSUNG VON GASBESTANDTEILEN DES ATEMGASES**
MEASUREMENT DEVICE THROUGH WHICH BREATHING GAS CAN FLOW FOR MEASURING GAS COMPONENTS OF THE BREATHING GAS
DISPOSITIF DE MESURE POUVANT ÊTRE TRAVERSÉ PAR UN GAZ RESPIRATOIRE POUR LA MESURE DES COMPOSANTS GAZEUX DU GAZ RESPIRATOIRE

(30) Priorität: 26.11.2018 DE 102018129838
(43) Veröffentlichungstag der Anmeldung: 06.10.2021
(73) Patentinhaber: Hamilton Medical AG, 7402 Bonaduz (CH)
(72) Erfinder: ROFFLER, Jürg, 7000 Chur (CH); BREITRUCK, Felix, 9470 Buchs (CH)
(74) Vertreter: Ruttensperger Lachnit Trossin Gomoll
(86) Internationale Anmeldenummer: PCT/EP2019/081992
(87) Internationale Veröffentlichungsnummer: WO 2020/109115

(56) Entgegenhaltungen:
- US-A- 3 725 658
- US-A1- 2006 251 903
- US-A1- 2016 184 545

## Beschreibung

Die vorliegende Erfindung betrifft eine Messvorrichtung zur Ermittlung wenigstens eines Gasbestandteils eines in einem Messraum der Messvorrichtung vorhandenen Gases, wobei die Messvorrichtung ein den Messraum umgebendes Gehäuse umfasst, von welchem wenigstens ein Gehäusewandabschnitt als Beobachtungsabschnitt zur Erfassung von vom Beobachtungsabschnitt in Richtung vom Messraum weg ausgehender elektromagnetischer Strahlung ausgebildet ist, wobei der Beobachtungsabschnitt wenigstens eine Folienlage umfasst und wobei das Gehäuse als Kunststoff-Spritzgussgehäuse ausgebildet ist.

Die vorliegende Erfindung betrifft außerdem ein Verfahren zur Herstellung eines Beobachtungsabschnitts einer solchen Messvorrichtung, wobei der Beobachtungsabschnitt den Messraum einer solchen Messvorrichtung wenigstens teilweise einfasst.

Messvorrichtungen der von der vorliegenden Anmeldung betroffenen Art sind im Stand der Technik und im einschlägigen Fachgebiet auch als "Messküvetten" oder "Messgasküvetten" bezeichnet. Sie dienen insbesondere in Beatmungsvorrichtungen dazu, Gasbestandteile, wie beispielsweise CO₂ oder/und O₂, in einem dem zu beatmenden Patienten zugeführten Inspirationsgas oder in einem von diesem ausgehenden Exspirationsgas anhand von elektromagnetischer Strahlung zu ermitteln, die für den jeweils zu untersuchenden Gasbestandteil charakteristisch ist bzw. die ein für den jeweils zu untersuchenden Gasbestandteil charakteristisches Strahlungsverhalten zeigt.

In der Ausführungsform der Fig. 5 der US 6,095,986 A zeigt die dort offenbarte bidirektional durchströmbare Messvorrichtung ein durch Spritzgießen erzeugtes Kunststoff-Basis-gehäuse. Die Beobachtungsabschnitte, durch welche in dem dort dargestellten Beispiel hindurch Infrarotstrahlung zur Messung des CO₂-Gehalts von Gas in der Messvorrichtung auf der einen Seite in den Messraum eingestrahlt wird und auf der anderen Seite nach Durchgang durch den gasgefüllten Messraum aus dem Messraum austritt, sind zunächst aus einem Basis-Gehäuse ausgespart.

Aus demselben Kunststoffmaterial wie das spritzgegossene Basis-Gehäuse sind Fensterbauteile spritzgegossen, die nach ihrer Herstellung auf eine infrarotstrahlungsdurchlässige Folie mit Antibeschlag-Beschichtung mittels Heißsiegeln aufgesiegelt werden. Die ausreichend infrarotstrahlungsdurchlässige Folie mit Antibeschlag-Beschichtung bedeckt eine das spritzgegossene Fensterbauteil durchsetzende Durchgangsöffnung desselben, so dass das Fensterbauteil zwar für Infrarotstrahlung durchlässig, jedoch für einen Gasaustausch zwischen Messraum und Außenumgebung undurchlässig ist.

Die so mit der Folie verbundenen Fensterbauteile werden anschließend in die oben genannten Aussparungen am Basis-Gehäuse eingesetzt und durch Verkleben oder Ultraschallschweißen mit diesem gasdicht verbunden.

Diese bekannte Messvorrichtung hat den Nachteil, dass zu ihrer Bildung eine Vielzahl von Verarbeitungsschritten notwendig ist, wobei besonders die stoffschlüssige Verbindung der Fensterbauteile mit dem Basis-Gehäuse sehr exakt ausgeführt sein muss, um tatsächlich die gewünschte Gasdichtigkeit bereitzustellen. Ebenso muss die Prozessführung beim Heißsiegeln der Fensterbauteile an die Folien sehr exakt eingestellt und überwacht werden, da zwischen den Fensterbauteilen und der Folie, auf die sie aufgesiegelt werden, erhebliche Dickenunterschiede bestehen, die eine Herstellung einer thermischen Schmelzverbindung erschweren.

Die US 2006/0251903 A1 offenbart ein Fenster für eine Gasanalysevorrichtung, etwa in Gestalt einer Messküvette, wobei das Fenster durch Spritzguss hergestellt und durch Verkleben oder Schweißen mit Ultraschall oder Hitze mit einem Beobachtungs-Spritzgusskörper verbunden wird. Die US 2006/0251903 A1 rät von der Verwendung von Folienlagen zur Bildung von Fenstern an Messküvetten ab.

Aus der US 3,725,658 sind eine Vorrichtung und Verfahren zur kontinuierlichen Erfassung von Sauerstoff in einem Gasstrom mittels eines fluoreszenten Materials bekannt.

Eine weitere Messvorrichtung zur Ermittlung eines Gasbestandteils in einem Gas ist aus der GB 2 533 806 B bzw. aus ihrem Familienmitglied US 2016/0184545 A1 bekannt. Diese Druckschrift, die sich insbesondere mit der Vermeidung von unerwünschten Leckagen an der Messvorrichtung befasst, weist auf die Leckage-Probleme hin, die gerade an den Kombinationsstellen von dünn- und dickwandigen Abschnitten entstehen. Die GB 2 533 806 B weist in diesem Zusammenhang explizit auf die Problemzone der fensterartigen Beobachtungsabschnitte mit dünnwandiger "Fensterscheibe" und verglichen damit dickwandigem "Fensterrahmen" zur Durchleuchtung des Messraums mit Infrarotstrahlen hin.

Die GB 2 533 806 B verwirft außerdem mehrstufige Spritzgussverfahren mit dem Hinweis, diese würden das Leckage-Problem von derartigen Messvorrichtungen nicht verbessern, da mehrstufige Spritzgussverfahren bewirkten, dass zu unterschiedlichen Zeiten spritzgusstechnisch hergestellte Bauteilabschnitte zu unterschiedlichen Zeiten erstarren, was wiederum an den Verbindungsstellen der zu unterschiedlichen Zeiten spritzgusstechnisch hergestellten Bauteilabschnitten zu Leckage-Problemen führen kann.

Die GB 2 533 806 B schlägt daher vor, die Messvorrichtung in einem einzigen Spritzguss-Schuss herzustellen und in dem so spritzgegossenen Bauteil Beobachtungsabschnitte zur Durchleuchtung des Messraums mit Infrarotstrahlung durch bewegliche Schieber nachzuformen.

Es werden daher bewegliche Schieber in die gerade mit Spritzgussmaterial gefüllte Spritzgusskavität eingefahren, bis am Ort der Schieber eine Wandstärke erreicht ist, die der gewünschten Wandstärke des Beobachtungsfensters entspricht.

Nachteilig an der Lösung der GB 2 533 806 B ist allerdings, dass diese das Problem der Aufnahme des Spritzgussmaterials ungelöst lässt, welches durch die in die Kavität eingefahrenen Schieber verdrängt werden muss. Spritzgusstechnisch muss die Kavität vollständig mit Spritzgussmaterial gefüllt sein, um ein Bauteil mit ausreichend definierter Gestalt in reproduzierbarer Bauteilqualität erzeugen zu können. Wenn dann jedoch Schieber in die gefüllte Kavität eingefahren werden, um Material in den Beobachtungsabschnitten zu verdrängen, muss dieses Material an anderer Stelle die Spritzgusskavität verlassen können. Dies führt zu Spritzgusswerkzeugen mit aufwendigen Nebenkavitäten, die gesteuert geöffnet und wieder geschlossen und entleert werden müssen.

Eine spritzgusstechnische Herstellung der Messvorrichtung in einem einzigen Spritzguss-Schuss mit bereits in die Kavität einragenden Schiebern ist technisch nicht ohne Weiteres möglich, da die Wandstärke der Beobachtungsfenster gemäß den Angaben in der GB 2 533 806 B 0,2 mm und vorzugsweise sogar 0,05 mm nicht übersteigen soll. Aufgrund der Erstreckungsfläche der Beobachtungsfenster mit dieser geringen Wandstärke kann der die Wandstärke definierende Kavitätsspalt beim Spritzgießen nicht sicher ausgefüllt werden.

Ein weiteres Problem der GB 2 533 806 B liegt in der infrarot-optischen Qualität der durch Schieber nach dem Spritzgießen erzeugten Beobachtungsfenster: Sind die Schieber thermisch nicht exakt auf die Prozessverhältnisse beim Spritzgießen abgestimmt, entziehen diese dem Spritzgussmaterial, das sie kontaktieren, lokal zu schnell oder zu langsam Wärme, so dass die von den Schiebern erzeugte Fensterfläche aufgrund von thermischem Verzug wenigstens abschnittsweise von der gewünschten ebenen Flächengestalt abweichen und somit zumindest abschnittsweise als Eintritts- bzw. Austrittslinse wirken kann. Somit kann Infrarotstrahlung, die auf einer Seite durch ein so gebildetes Fenster in den Messraum eingestrahlt wird und auf der anderen Seite durch ein gegenüberliegendes Fenster wieder austritt, unter Umständen mehrfach gebrochen, abgelenkt oder/und lokal gebündelt werden, was die Qualität des außerhalb des Messraums am Beobachtungsabschnitt sensorisch erhaltenen Infrarotstrahlungssignals beeinträchtigen kann.

Eine weitere Messvorrichtung ist aus der DE 10 2006 052 999 A1 bekannt. Auch diese als "Messgasküvette" bezeichnete Messvorrichtung dient, wie die vorgenannten Messvorrichtungen, der Erfassung des CO₂-Anteils im Atemgas durch an sich bekannte Infrarot-Spektroskopie bzw. Kapnometrie.

Die aus der DE 10 2006 052 999 A1 bekannte Messvorrichtung umfasst zwei gesondert hergestellte Spritzgusskörper. Bei dessen Herstellung wird ein erster dünnwandiger innerer Spritzgusskörper mit einem äußeren dickwandigeren weiteren Spritzgusskörper umspritzt. Dabei werden auf gegenüberliegenden Seiten des inneren Spritzgusskörpers durch Schieber Bereiche belegt, in welchen Spritzgussmaterial des zweiten äußeren Körpers nicht an den ersten Körper gelangen können soll. So werden im zweiten Spritzgussschritt Beobachtungsfenster durch Schieber am äußeren Spritzgusskörper ausgespart. Die ausgesparten Fenster sind durch das Material des umspritzten inneren Spritzgusskörpers bedeckt und somit verschlossen.

Mit dem aus der DE 10 206 052 999 A1 bekannten Verfahren sollen am inneren, die Fensterflächen der Beobachtungsfenster bildenden Spritzgusskörper Wandstärken im Bereich von 170 bis 210 µm realisiert werden können.

Nachteilig an dieser bekannten Messvorrichtung und ihrem Herstellungsverfahren ist die äußerst schwierige spritzgusstechnische Herstellung des dünnwandigen inneren Spritzgusskörpers. Diese erfordert vorrichtungsmäßig eine thermisch hinsichtlich ihrer Wärmekapazität und Wärmeleitung exakt austarierte Spritzgussform und erfordert prozesstechnisch einen erheblichen Aufwand, bis die entsprechende Spritzgussform thermisch so im Gleichgewicht ist, dass der wie angegeben dünnwandige und im Verhältnis zu seiner Wandstärke großflächige Spritzgusskörper mit einigermaßen Sicherheit wiederholbar erzeugt werden kann.

Es ist im Hinblick auf den oben dargelegten Stand der Technik Aufgabe der vorliegenden Erfindung, eine technische Lehre anzugeben, mit welcher eine Messvorrichtung der eingangs genannten Art sicher mit möglichst geringem Aufwand wiederholgenau und ohne unerwünschte Leckagen herstellbar ist.

Gemäß der vorliegenden Erfindung wird diese Aufgabe gelöst durch eine gattungsgemäße Messvorrichtung, die in Anspruch 1 definiert ist.

Durch die Verwendung der wenigstens einen Folienlage kann im Beobachtungsabschnitt der Messvorrichtung ein sehr dünner Wandbereich sicher und wiederholgenau bereitgestellt werden. Ein Folienkörper, umfassend eine Folienlage oder eine Mehrzahl von laminierten oder/und coextrudierten Folienlagen, kann dabei dünner als ein Spritzgusskörper hergestellt werden. Darüber hinaus kann insbesondere eine Mehrzahl von Folienlagen einen mehrlagig ausgebildeten Schicht- bzw. Folienkörper mit abhängig von den verwendeten Folienlagen in Grenzen einstellbaren Körpereigenschaften bilden.

Durch das Anspritzen des Beobachtungs-Spritzgusskörpers an die wenigstens eine Folienlage wird eine sichere stoffschlüssige Verbindung des Beobachtungs-Spritzgusskörpers mit der wenigstens einen Folienlage bereits beim Füllen der Spritzgusskavität zur Herstellung des Beobachtungs-Spritzgusskörpers gebildet, also dann, wenn das Material des späteren Beobachtungs-Spritzgusskörpers noch fließfähig und warm bzw. heiß ist. Ein Heißsiegelschritt wie in der US 6,095,986 A zur Verbindung von Folienlage und Beobachtungs-Spritzgusskörper kann somit entfallen. Es ist überdies, im Gegensatz zu dem bekannten Aufsiegeln, keine äußere Wärmequelle notwendig, um eine ausreichend dichte Verbindung des Beobachtungs-Spritzgusskörpers mit der wenigstens einen Folienlage herzustellen. Durch den Einspritzdruck beim Einspritzen des Materials zur Bildung des Beobachtungs-Spritzgusskörpers wird außerdem über die notwendige Wärmemenge zur Bildung einer gasdichten stoffschlüssigen Verbindung des Beobachtungs-Spritzgusskörpers mit der wenigstens einen Folienlage hinaus auch die notwendige Kraft zur Bildung einer solchen Verbindung bereitgestellt.

Das so durch Anspritzen des Beobachtungs-Spritzgusskörpers an die wenigstens eine Folienlage gebildete Beobachtungs-Wandbauteil kann dann in einem weiteren Spritzgussprozess mit dem Rahmen-Spritzgusskörper zu dem Gehäuse der Messvorrichtung ergänzt werden. Hierzu ist der wenigstens eine bereits hergestellte Beobachtungs-Spritzgusskörper einschließlich der mit ihm verbundenen Folienlage in die Kavität zur Herstellung des Rahmen-Spritzgusskörpers einzulegen, so dass das Beobachtungs-Wandbauteil, insbesondere der Beobachtungs-Spritzgusskörper, vom fließfähigen Material des Rahmen-Spritzgusskörpers benetzt und aufgrund der thermischen Energie sowie des hohen Drucks des Rahmen-Spritzgussmaterials beim Einspritzen mit dem Rahmen-Spritzgusskörper verbunden werden kann.

So kann mit der Herstellung des Rahmen-Spritzgusskörpers eine stoffschlüssige Verbindung zwischen dem wenigstens einen Beobachtungs-Wandbauteil, insbesondere Beobachtungs-Spritzgusskörper, und dem Rahmen-Spritzgusskörper hergestellt werden, die vollständig gasdicht ist.

Da mit der wenigstens einen Folienlage bereits ein dünnwandiger Bereich des Beobachtungsabschnitts bereitgestellt ist, kann der Beobachtungs-Spritzgusskörper nahezu beliebig dick ausgeführt werden, so dass eine ausreichende Benetzungsfläche zwischen dem an das Beobachtungs-Wandbauteil, insbesondere an den Beobachtungs-Spritzgusskörper, angespritzten Rahmen-Spritzgusskörper und dem Beobachtungs-Wandbauteil zur Herstellung einer dichten stoffschlüssigen Verbindung bereitgestellt werden kann.

Eine beim Anspritzen des Rahmen-Spritzgusskörpers an den Beobachtungs-Spritzgusskörper vom Material des Rahmen-Spritzgusskörpers benetzte Fläche des Beobachtungs-Spritzgusskörpers kann von einer ebenen Gestalt abweichen, um die Größe der vom Material des Rahmen-Spritzgusskörpers benetzten Fläche des Beobachtungs-Spritzgusskörpers zu erhöhen, ohne dessen Wandstärke erhöhen zu müssen. Beispielsweise kann die benetzte Fläche eine, vorzugsweise entlang ihrer Längserstreckung verlaufende, Nut oder einen, vorzugsweise entlang ihrer Längserstreckung verlaufenden, Vorsprung aufweisen. Dann kann zusätzlich zur stoffschlüssigen Verbindung auch eine formschlüssige Verbindung zwischen dem Rahmen-Spritzgusskörper und dem Beobachtungs-Spritzgusskörper erzeugt werden, was die Verbindungsfestigkeit zwischen den genannten Körpern zusätzlich erhöht.

Entsprechend dem oben Gesagten wird die oben genannte Aufgabe ebenfalls gelöst durch ein Verfahren nach Anspruch 14, wobei das Verfahren die folgenden Schritte umfasst:
- Einlegen eines Folienkörpers mit wenigstens einer Folienlage in eine Spritzgusskavität,
- Anspritzen eines Beobachtungs-Spritzgusskörpers an den Folienkörper und dadurch Bilden eines Beobachtungs-Wandbauteils,
- Anspritzen eines zweiten Spritzguss-Kunststoffkörpers an das Beobachtungs-Wandbauteil und dadurch Herstellen des Beobachtungsabschnitts.

Das Verfahren dient zur Herstellung wenigstens eines Beobachtungsabschnitts, also eines Abschnitts eines Gehäuses einer oben beschriebenen Messvorrichtung, welche einen Messraum wenigstens teilweise einfasst. Der Beobachtungsabschnitt ist zur Beobachtung und Erfassung von von ihm ausgehenden elektromagnetischen Strahlen an der Messvorrichtung geeignet.

Selbstverständlich soll nicht ausgeschlossen sein, dass mit dem oben beschriebenen Verfahren das gesamte Gehäuse einer Messvorrichtung und somit eine gesamte Einfassung eines Messraums hergestellt wird. Dies ist sogar bevorzugt. Erfindungsgemäß reicht es aber zur Verwirklichung der Vorteile der vorliegenden Erfindung bereits aus, wenn durch das Verfahren jener Abschnitt des Messvorrichtungsgehäuses hergestellt wird, der die Beobachtung und Erfassung von vom Beobachtungsabschnitt der Messvorrichtung ausgehenden elektromagnetischen Strahlen ermöglicht. Wie dieser Beobachtungsabschnitt zu einem Messvorrichtungsgehäuse ergänzt wird, ist dann unerheblich. Dabei ist es bevorzugt, wenn der zweite Spritzguss-Kunststoffkörper der oben genannte Rahmen-Spritzgusskörper ist und somit mit zwei Spritzguss-Vorgängen eine funktionsfähige Messvorrichtung gebildet werden kann.

Grundsätzlich können im Mehr-Komponenten-Spritzgussverfahren in einem einzigen Spritzguss-Formwerkzeug sowohl der Beobachtungs-Spritzgusskörper an die wenigstens eine Folienlage angespritzt als auch anschließend der zweite Spritzguss-Kunststoffkörper an den Beobachtungs-Spritzgusskörper bzw. an das Beobachtungs-Wandbauteil angespritzt werden. Bereiche der Spritzgusskavität, die im jeweiligen Spritzgussschritt gerade nicht von Spritzgussmaterial erreicht werden sollen, können durch Schieber belegt sein.

Eine größere konstruktive Freiheit erhält man jedoch, wenn der Folienkörper in eine erste Spritzgusskavität eingelegt wird, in welcher der Beobachtungs-Spritzgusskörper an den Folienkörper angespritzt und so das Beobachtungs-Wandbauteil gebildet wird, und wenn anschließend das Beobachtungs-Wandbauteil in einer von der ersten verschiedenen zweiten Spritzgusskavität angeordnet wird, in welcher dann der zweite Spritzguss-Kunststoffkörper an das wenigstens eine Beobachtungs-Wandbauteil angespritzt wird.

Nachfolgend werden die Vorrichtung und das Verfahren gemeinsam erläutert und weitergebildet.

Grenzflächen des Beobachtungs-Wandbauteils bilden in der, vorzugsweise zweiten, Spritzgusskavität zur Herstellung des zweiten Spritzguss-Kunststoffkörpers Grenzflächen dieser Spritzgusskavität.

Eine ausreichend strapazierfähige und für elektromagnetische Strahlung im relevanten Wellenlängenbereich durchlässige Folienlage ist bevorzugt aus biaxial orientiertem Polymer gebildet. Dabei ist biaxial orientiertes Polyolefin besonders bevorzugt. In der Gruppe der Polyolefine ist biaxial orientiertes Polypropylen (BOPP) wegen seiner Strahlungsdurchlässigkeit und seiner hohen mechanischen Festigkeit bei bereits geringen Wandstärken bevorzugt. Bevorzugt umfasst daher die wenigstens eine Folienlage, an welche der Beobachtungs-Spritzgusskörper angespritzt wird, BOPP. Dies schließt nicht aus, dass der Folienkörper auf der vom Beobachtungs-Spritzgusskörper abgewandten Seite der BOPP-Folienlage oder/und auf der dem Beobachtungs-Spritzgusskörper zugewandten Seite weitere Folienlagen oder Schichten, wie etwa Lackschichten, aufweist, beispielsweise um die Beschlagneigung des Folienkörpers in Kontakt mit feuchtem Gas herabzusetzen.

Dann, wenn der Folienkörper mehr als eine Folienlage aufweist, kann dieser bevorzugt eine Schutzfolienlage, insbesondere aus Polycarbonat, aufweisen. Bevorzugt ist die Polycarbonat-Folienlage auf der dem Beobachtungs-Spritzgusskörper zugewandten Seite einer Polyolefin-Folienlage, wie etwa der oben erwähnten besonders bevorzugten BOPP-Folienlage, angeordnet. Der Beobachtungs-Spritzgusskörper kann dann unmittelbar an die Schutzfolienlage angespritzt sein, insbesondere an eine Schutzfolienlage aus Polycarbonat. Die Schutzfolienlage kann im Verbund mit der BOPP-Folienlage die Formstabilität des Folienverbundes erhöhen, was gerade unter den beim Anspritzen herrschenden thermischen Belastungen sowie mechanischen Belastungen durch Schwund beim Abkühlen von Vorteil ist.

Vorzugsweise ist die Schutzfolienlage, die nicht unbedingt aus Polycarbonat hergestellt sein muss, jedoch bevorzugt die oben genannte Polycarbonat-Folienlage ist, dicker als die Polyolefin-, insbesondere BOPP-Folienlage. Bevorzugt ist die aus Polycarbonat hergestellte Schutzfolie wenigstens 4 bis 7 mal so dick wie die BOPP-Folienlage, besonders bevorzugt 5,5 bis 6,5 mal so dick wie die BOPP-Folienlage.

Die BOPP-Folienlage kann eine Dicke im Bereich von 30 bis 70 µm aufweisen, bevorzugt im Bereich von 35 bis 50 µm. In Versuchen hat sich eine BOPP-Folienlage mit einer Dicke im Bereich von 40 bis 45 µm bewährt, wobei nach augenblicklichem Kenntnisstand eine Dicke von 41 µm besonders bevorzugt ist.

Die Schutzfolie, insbesondere aus Polycarbonat, kann eine Dicke im Bereich von 100 bis 300 µm aufweisen, wobei ein Dickenbereich von 230 bis 270 µm bevorzugt ist. In Versuchen hat sich eine Polycarbonat-Folienlage mit einer Dicke im Bereich von 245 bis 255 µm bewährt, wobei nach derzeitigem Kenntnisstand eine Lagendicke von 250 µm für die Schutzfolie bevorzugt ist.

Zur dauerhaften Verbindung der Polyolefin-Folienlage, insbesondere BOPP-Folienlage, mit der Schutzfolienlage, insbesondere Polycarbonat-Folienlage, kann zwischen den beiden Folienlagen eine haftvermittelnde Schicht angeordnet sein. Bevorzugt kann Acrylat-Klebstoff, insbesondere Reinacrylat-Klebstoff, zwischen der BOPP-Folienlage und der Polycarbonat-Folienlage als haftvermittelnde Schicht angeordnet sein. Eine solche Schicht kann als Acrylat-Klebstofffolie oder Reinacrylat-Klebstofffolie, etwa mit einer Dicke von 60 bis 120 µm, besonders bevorzugt mit einer Dicke von 100 µm, als haftvermittelnde Folie zwischen der BOPP-Folienlage und der Schutzfolienlage angeordnet sein.

Der Beobachtungs-Spritzgusskörper ist vorzugsweise aus einem Kunststoff auf Basis von Acrylnitrilbutadienstyrol (ABS) gebildet. Besonders bevorzugt ist der zur Bildung des Beobachtungs-Spritzgusskörpers verwendete Kunststoff transparentes ABS, also beispielsweise Methylmetacrylat-Arcrylnitrylbutadienstyrol (MABS). Dieses verbindet sich beim Spritzgussvorgang hervorragend mit der oben genannten BOPP-Folienlage oder mit der oben genannten Polycarbonat-Folienlage und gestattet obendrein die visuelle Überprüfung des Messraums.

Zur Herstellung einer möglichst gasdichten Messvorrichtung mit möglichst guter Verbindung zwischen Beobachtungs-Wandbauteil, insbesondere Beobachtungs-Spritzgusskörper, und Rahmen-Spritzgusskörper ist der Rahmen-Spritzgusskörper bevorzugt aus demselben Material gebildet wie der Beobachtungs-Spritzgusskörper.

Grundsätzlich kann beliebig im Messraum vorhandenes Gas mit der vorliegend diskutierten Messvorrichtung hinsichtlich bestimmter Gasbestandteile untersucht werden. Zur Verwendung der Messvorrichtung zur Analyse von strömenden Gasen ist der Messraum bevorzugt längs einer virtuellen Strömungsbahn von Gas durchströmbar, besonders bevorzugt bidirektional, so dass sowohl inspiratorisches als auch exspiratorisches Atemgas analysiert werden kann. Der bevorzugte Anwendungsfall der vorliegenden Messvorrichtung liegt in der Anordnung in einer Atemgasleitung einer Beatmungsvorrichtung und somit in der Analyse von inspiratorischem und exspiratorischem Atemgas eines künstlich beatmeten Patienten.

Eine mögliche Analyse der vom Beobachtungsabschnitt ausgehenden elektromagnetischen Strahlung dient der Ermittlung des im beobachteten Gas enthaltenen CO₂ (Kohlendioxid). Hierzu wird in an sich bekannter Weise Infrarotstrahlung verwendet, die von einer Infrarot-Strahlenquelle derart in den Messraum eingestrahlt wird, dass sie im Bereich des wenigstens einen Beobachtungsabschnitts außerhalb des Messvorrichtungsgehäuses erfassbar ist. Vorteilhafterweise ist in einem zur CO₂-Ermittlung im beobachteten Gas ausgebildeten Beobachtungsabschnitt die wenigstens eine Folienlage der einzige Festkörper, welcher von der Infrarotstrahlung oder allgemein von der verwendeten elektromagnetischen Strahlung durchdrungen werden muss. Daher ist es für diese mögliche Ausgestaltung der Messvorrichtung vorteilhaft, dass der Beobachtungs-Spritzgusskörper eine Beobachtungs-Aussparung aufweist, innerhalb welcher die wenigstens eine Folienlage zugänglich ist. Die Beobachtungs-Aussparung durchsetzt daher bevorzugt den Beobachtungs-Spritzgusskörper und ist von der wenigstens einen Folienlage bedeckt. Die wenigstens eine Folienlage bildet in diesem Fall eine Fensterfläche, wie sie aus dem Stand der Technik bekannt ist, der Beobachtungs-Spritzgusskörper bildet einen "Fensterrahmen".

Um das Beobachtungs-Wandbauteil möglichst passgenau in der Spritzgusskavität anordnen zu können, in welcher der zweite Kunststoff-Spritzgusskörper, insbesondere der Rahmen-Spritzgusskörper, unter gleichzeitigem Anspritzen an das Beobachtungs-Wandbauteil erzeugt wird, kann vorgesehen sein, dass eine Randfläche der Beobachtungs-Aussparung sich in Richtung zur wenigstens einen Folienlage hin verjüngt und so als Zentrierfläche für einen Angriff eines Werkzeugs, insbesondere Zentrierwerkzeugs, ausgebildet ist. Der Werkzeugeingriff kann von einem Bauteil des Formwerkzeugs ausgehen, welches die Spritzgusskavität des Rahmen-Spritzgusskörpers definiert, etwa von einem Formkern oder einem Schieber. Die Beobachtungs-Aussparung kann eine polyedrische oder allgemein nicht-rotationssymmetrische Randfläche aufweisen, so dass mit der Zentrierung auch eine rotatorische Ausrichtung um eine die Beoachtungs-Aussparung durchsetzende virtuelle Aussparungsachse möglich ist. Grundsätzlich ist jedoch auch eine sich konisch verjüngende Beobachtungs-Aussparung möglich. Zur Ermittlung des CO₂-Anteils in dem zu beobachtenden Gas im Messraum ist die wenigstens eine Folienlage bevorzugt für elektromagnetische Strahlung im infraroten Spektralbereich durchlässig.

Grundsätzlich ist es möglich, dass eine Strahlenquelle, beispielsweise eine Infrarot-Strahlenquelle, im Messraum angeordnet ist und von dort aus das im Messraum befindliche Gas sowie den von der Strahlenquelle aus betrachtet hinter dem Gas liegenden Beobachtungsabschnitt durchstrahlt. In diesem Fall reicht es aus, wenn die Messvorrichtung einen einzigen Beobachtungsabschnitt bzw. einen einzigen Beobachtungs-Spritzgusskörper mit wenigstens einer Folienlage aufweist.

Gerade bei der Ermittlung des CO₂-Gehalts des Gases im Messraum ist es jedoch vorteilhaft, wenn das Gas im Messraum möglichst ungestört von einer im Betrieb auch Wärme entwickelnden Strahlenquelle im Inneren des Messraums ist. Bevorzugt wird daher der Messraum mit Infrarotstrahlung oder allgemein mit elektromagnetischer Strahlung durchleuchtet. Um den Messraum mit elektromagnetischer Strahlung durchleuchtbar auszugestalten, kann vorgesehen sein, dass die Messvorrichtung ein erstes und ein zweites Beobachtungs-Wandbauteil umfasst, welche zur Erfassung eines ersten Gasbestandteils derart am Gehäuse vorgesehen sind, dass sich wenigstens ein Abschnitt des Messraums zwischen ihnen befindet. Zur Unterscheidung dieser Beobachtungs-Wandbauteile von anderen Beobachtungs-Wandbauteilen, die zur Erfassung eines anderen, zweiten Gasbestandteils ausgebildet oder/und vorgesehen sind, sind die vorliegend diskutierten Beobachtungs-Wandbauteile als "Erster-Gasbestandteil-Beobachtungs-Wandbauteile" bezeichnet.

Das erste und das zweite Erster-Gasbestandteil-Beobachtungs-Wandbauteil sind dabei derart am Gehäuse angeordnet, dass elektromagnetische Strahlung, insbesondere Licht, besonders bevorzugt Infrarotstrahlung, welche durch einen Beobachtungsabschnitt, der einen der beiden Erster-Gasbestandteil-Beobachtungs-Wandbauteile umfasst, in den Messraum eingestrahlt wird und durch den Beobachtungsabschnitt des jeweils anderen Erster-Gasbestandteil-Beobachtungs-Wandbauteils aus dem Messraum wieder ausstrahlen kann.

Bei der Ermittlung von Gasbestandteilen eines Gases im Messraum kann die Temperatur des zu beobachtenden Gases eine wichtige Rolle spielen.

Die Messvorrichtung kann zusätzlich oder alternativ zum oben genannten Beobachtungsabschnitt, welcher für eine Durchstrahlung des Gases im Messraum mit elektromagnetischer Strahlung ausgebildet ist, einen Beobachtungsabschnitt zur Ermittlung der Gastemperatur aufweisen. Hierzu kann die wenigstens eine Folienlage eines so gebildeten Temperatur-Beobachtungsabschnitts als Temperaturmess-Folienlage eine Metallfolie umfassen. Die Metallfolie, die in der Regel eine Dicke von weniger als 20 µm, bevorzugt von weniger als 10 µm aufweist, ist zum einen gasdicht und weist zum anderen, verglichen mit Kunststofffolien, eine hohe Wärmeleitfähigkeit auf. Bevorzugt ist die Metallfolie eine gegenüber Oxidation unanfällige, leicht verfügbare und sehr gut wärmeleitende Aluminiumfolie. Die Temperaturmess-Folienlage und der daran angespritzte Beobachtungs-Spritzgusskörper bilden ein Temperatur-Beobachtungs-Wandbauteil.

Zur besseren Erfassung der von der Metallfolie der Temperaturmess-Folienlage abgestrahlten Wärmestrahlung kann diese im Beobachtungsabschnitt auf ihrer vom Messraum weg weisenden Seite mit schwarzem Material, etwa schwarzem Lack oder schwarzem Kunststoff, beschichtet sein. Die so gebildete Temperaturmess-Folienlage wirkt bei Betrachtung von außerhalb des Messraums nahezu wie ein schwarzer Strahler, so dass aufgrund der geringen Dicke der Metallfolie und ihrer hohen Wärmeleitfähigkeit durch Erfassung der von der schwarzen Beschichtung ausgehenden Wärmestrahlung sehr genau auf die Temperatur des Gases im Messraum geschlossen werden kann.

Die Temperaturmess-Folienlage kann, wie die oben beschriebene Folienlage aus vorzugsweise BOPP, von außerhalb des Messraums, d. h. von außerhalb der Messvorrichtung, durch eine Beobachtungs-Aussparung zugänglich sein. Es gilt das oben Gesagte auch hier: die Beobachtungs-Aussparung kann den Beobachtungs-Spritzgusskörper in Dickenrichtung durchsetzen und von der Temperaturmess-Folienlage abgedeckt sein. Der Rand der Beobachtungs-Aussparung kann, wie oben beschrieben, zur exakten Anordnung des Temperatur-Beobachtungs-Wandbauteils in einer weiteren Spritzguss-Kavität zur Temperaturmess-Folienlage hin verjüngt ausgebildet sein.

Das oben beschriebene Temperatur-Beobachtungs-Wandbauteil kann also zusätzlich oder alternativ zu der bevorzugt aus BOPP gebildeten Kunststofffolie die beschriebene Temperaturmess-Folienlage aufweisen. Sofern das die Temperaturmess-Folienlage aufweisende Temperatur-Beobachtungs-Wandbauteil auch eine, insbesondere aus BOPP gebildete, Kunststofffolie aufweist, ist diese bevorzugt zwischen dem Beobachtungs-Spritzgusskörper und der Temperaturmess-Folienlage angeordnet. Zur Erleichterung eines Anspritzens des Beobachtungs-Spritzgusskörpers an die bevorzugt metallische Temperaturmess-Folienlage kann die Temperaturmess-Folienlage eine Primerschicht aufweisen oder vor dem Spritzgießen mit der Kunststofffolie zu einem Schichtkörper laminiert sein. Der Beobachtungs-Spritzgusskörper wird dann an die Kunststofffolienlage des Schichtkörpers angespritzt. Zur möglichst geringen Beeinflussung der von der Temperaturmess-Folienlage ausgehenden Wärmestrahlung ist die Kunststofffolie bevorzugt im Bereich der Beobachtungs-Aussparung ausgespart.

Wenngleich der eingangs genannte Stand der Technik sich ausschließlich auf die Messung des CO₂-Gehalts in einem Gas bezieht, ist es keineswegs so, dass CO₂ der einzige Gasbestandteil ist, der durch Beobachtung oder/und Erfassung einer von einem Beobachtungsabschnitt ausgehenden elektromagnetischen Strahlung erfassbar ist. Zusätzlich oder alternativ kann zur Erfassung eines vom ersten verschiedenen zweiten Gasbestandteils vorgesehen sein, dass die wenigstens eine Folienlage als Zweiter-Gasbestandteil-Folienlage zur Erfassung des zweiten Gasbestandteils eine Fotolumineszenz-Lage mit wenigstens einem darin aufgenommenem Luminophor aufweist.

Bestimmte Gasbestandteile, wie beispielsweise O₂ (Sauerstoff), haben die Eigenschaft, die Strahlung eines vom Gas benetzten und durch eine externe Strahlenquelle zum Strahlen angeregten Luminophors abhängig von ihrer Konzentration im Gas abzulöschen. Durch dieses so genannte "Quenching" ändert sich die Dauer oder/und die Intensität der Strahlung des durch die externe Strahlenquelle zum Strahlen angeregten Luminophors in der Fotolumineszenz-Lage in Abhängigkeit von der Konzentration des mit dem Luminophor in Kontakt tretenden ablöschenden Gasbestandteils.

Die Bezeichnung erster Gasbestandteil und zweiter Gasbestandteil dienen hier lediglich der Unterscheidung der Gasbestandteile. Die Messvorrichtung kann nur die Zweiter-Gasbestandteil-Folienlage aufweisen und somit nur zur Ermittlung des zweiten Gasbestandteils durch Fotolumineszenz ausgebildet sein.

Da gerade für fotolumineszenzbasierte Verfahren zur Ermittlung eines Gasbestandteils eines zu beobachtenden Gases dessen Temperatur eine große Rolle spielt, weist die zur Ermittlung des zweiten Gasbestandteils ausgebildete Messvorrichtung bevorzugt ein Zweiter-Gasbestandteil-Beobachtungs-Wandbauteil mit der Zweiter-Gasbestandteil-Folienlage auf, welcher auch die Temperaturmess-Folienlage aufweist.

Um zu verhindern, dass die Strahlung der Fotolumineszenz-Lage von einem Gasbestandteil der die Messvorrichtung außerhalb des Messraums umgebenden Atmosphäre beeinflusst wird, kann die Zweiter-Gasbestandteil-Folienlage auf ihrer vom Messraum abgewandten Seite vollständig vom Beobachtungs-Spritzgusskörper bedeckt sein. Damit das Signal der Fotolumineszenz-Lage auswertbar ist, ist der Beobachtungs-Spritzgusskörper des Zweiter-Gasbestandteil-Beobachtungs-Wandbauteils für elektromagnetische Strahlung im Wellenlängenbereich der von der Fotolumineszenz-Lage abgestrahlten Fotolumineszenz-Strahlung durchlässig. Da die Fotolumineszenz-Lage nicht nur elektromagnetische Strahlung in einem vorbestimmten Wellenlängenbereich abstrahlt, sondern auch durch elektromagnetische Strahlung in einem vom abgestrahlten Wellenlängenbereich verschiedenen Anregungswellenlängenbereich zum Strahlen angeregt werden muss, ist der Beobachtungs-Spritzgusskörper bevorzugt auch für elektromagnetische Strahlung im Wellenlängenbereich der die Fotolumineszenz-Lage anregenden Anregungsstrahlung durchlässig, um diese Anregung von außerhalb des Messraums zu ermöglichen.

Dadurch, dass der Beobachtungs-Spritzgusskörper nahezu mit beliebiger Wanddicke ausgebildet werden kann, kann sichergestellt sein, dass ein in der die Messvorrichtung umgebenden Atmosphäre vorhandener zweiter Gasbestandteil, beispielsweise Sauerstoff, den Beobachtungs-Spritzgusskörper nicht bis zur Fotolumineszenz-Lage durchdringt, so dass die zu beobachtende Ablöschung der abgestrahlten Fotolumineszenz-Strahlung ausschließlich auf dem zweiten Gasbestandteil im Gas im Messraum beruht.

Bevorzugt ist die Messvorrichtung sowohl zur Ermittlung des ersten Gasbestandteils als auch zur Ermittlung des zweiten Gasbestandteils ausgebildet. Hierzu kann die Messvorrichtung sowohl das erste und das zweite Erster-Gasbestandteil-Beobachtungs-Wandbauteil als auch ein Zweiter-Gasbestandteil-Beobachtungs-Wandbauteil mit der Zweiter-Gasbestandteil-Folienlage aufweisen.

Zur Bildung einer räumlich kompakten Messvorrichtung, die dennoch zwei unterschiedliche Gasbestandteile im Gas im Messraum ermitteln kann, sind am Gehäuse das erste und das zweite Erster-Gasbestandteil-Beobachtungs-Wandbauteil zu je einer anderen Seite des Zweiter-Gasbestandteil-Beobachtungs-Wandbauteils angeordnet. Dann, wenn, wie bevorzugt, der Messraum längs der oben genannten Strömungsbahn von Gas durchströmbar ist, überlappen sich längs der Strömungsbahn bevorzugt die beiden Erster-Gasbestandteil-Beobachtungs-Wandbauteile und das Zweiter-Gasbestandteil-Beobachtungs-Wandbauteil wenigstens teilweise, vorzugsweise vollständig.

Zur Verwendung in einem Gasleitungssystem ist bevorzugt an wenigstens einem Längsende des Gehäuses eine Anschlussformation angeordnet, welche zum Anschluss einer Schlauch- oder/und Rohrleitung ausgebildet ist. Da das Gehäuse spritzgusstechnisch hergestellt ist, ist die Anschlussformation vorzugsweise einstückig mit einem den Messraum einfassenden Beobachtungsbereich der Messvorrichtung ausgebildet. Der Beobachtungsbereich umfasst eine Mehrzahl von Beobachtungsabschnitten, vorzugsweise alle Beobachtungsabschnitte.

Bevorzugt ist an beiden Längsenden des Gehäuses je eine solche Anschlussformation angeordnet. Die Anschlussformation ist insbesondere zum Anschluss einer Atemgasleitung einer Beatmungsvorrichtung ausgebildet.

Die vorliegende Beschreibung betrifft außerhalb des Schutzbereichs der vorliegenden Erfindung auch eine Messvorrichtung zur Ermittlung wenigstens eines Gasbestandteils eines in einem Messraum der Messvorrichtung vorhandenen Gases, wobei die Messvorrichtung ein den Messraum umgebendes Gehäuse umfasst, von welchem wenigstens ein Gehäusewandabschnitt als Beobachtungsabschnitt zur Erfassung von vom Beobachtungsabschnitt in Richtung vom Messraum weg ausgehender elektromagnetischer Strahlung ausgebildet ist, wobei der Beobachtungsabschnitt wenigstens ein Fensterbauteil umfasst, welches wenigstens teilweise, vorzugsweise vollständig, aus für die elektromagnetische Strahlung durchlässigem Glas gebildet ist und wobei das Gehäuse als Kunststoff-Spritzgussgehäuse ausgebildet ist.

Eine solche Messvorrichtung kann sicher mit möglichst geringem Aufwand wiederholgenau und ohne unerwünschte Leckagen dadurch herstellbar sein, dass der Beobachtungsabschnitt wenigstens ein Beobachtungs-Wandbauteil aufweist, wobei das Beobachtungs-Wandbauteil einen Beobachtungs-Spritzgusskörper umfasst, von welchem wenigstens ein Abschnitt des Fensterbauteils umspritzt ist.

Das Fensterbauteil kann wegen der Durchlässigkeit für Infrarotstrahlung dann zur Ermittlung des CO₂-Anteils des Gases verwendet werden, wenn das Glas des Fensterbauteils aus Aluminiumoxid oder aus Germanium gebildet ist. Das Fensterbauteil kann daher Saphirglas oder Chalkogenidglas umfassen.

Für eine ebenso gasdichte wie mechanisch feste Verbindung des Fensterbauteils mit dem Beobachtungs-Spritzgusskörper kann das Fensterbauteil bevorzugt längs seines gesamten Außenumfangs von Material des Beobachtungs-Spritzgusskörpers umspritzt sein. Zu dem Zweck einer ebenso festen wie dichten Anordnung des Fensterbauteils in einer Beobachtungs-Aussparung des Beobachtungs-Spritzgusskörpers kann das Fensterbauteil an seinem Außenumfang wenigstens längs eines Umfangsabschnitts, vorzugsweise längs seines gesamten Außenumfangs, einen von einem radial weiter innen gelegenen Fensterabschnitt nach radial außen abstehenden Ankervorsprung aufweisen.

Der Ankervorsprung kann in Dickenrichtung dünner ausgebildet sein als der Fensterabschnitt, von dem er nach radial außen absteht. Dies ermöglicht die bevorzugte Anordnung des von Material des Beobachtungs-Spritzgusskörpers umspritzten Fensterbauteils derart, dass die zum Messraum hinweisende Innenseite des Fensterbauteils bzw. des Fensterabschnitts bündig mit der Innenfläche des das Fensterbauteil umgebenden Beobachtungs-Spritzgusskörpers angeordnet ist. So kann zum einen in Dickenrichtung beiderseits des Ankervorsprungs Material des Beobachtungs-Spritzgusskörpers vorhanden sein, was das Fensterbauteil fest im Beobachtungs-Spritzgusskörper verankert. Zum anderen kann ein Beobachtungsabschnitt mit im Wesentlichen glatter, vorsprungsfreier Innenfläche erhalten werden, sodass eine unerwünschte Ansammlung von Feuchtigkeit im Übergangsbereich zwischen Beobachtungs-Spritzgusskörper und Fensterbauteil vermieden werden kann.

Der Ankervorsprung kann bündig mit der Außenfläche des Fensterabschnitts ausgebildet sein und sich an der Außenfläche stufenlos an den Fensterabschnitt anschließen. Eine Stufe zwischen dem Ankervorsprung und dem Fensterabschnitt liegt dann ausschließlich auf der Innenseite des Fensterbauteils vor, wo der Ankervorsprung bezüglich der Innenfläche des Fensterabschnitts vorteilhaft in Dickenrichtung zurückgesetzt ist. Hierdurch kann bei stabilem Ankervorsprung und bei bündiger Anordnung der Innenfläche des Fensterabschnitts und der Innenfläche des Beobachtungs-Spritzgusskörpers eine maximale Menge an Material des Beobachtungs-Spritzgusskörpers zwischen dem Ankervorsprung und der Innenfläche des Beobachtungs-Spritzgusskörpers bzw. des Messraums angeordnet werden. Dies erhöht die Festigkeit der Verbindung von Beobachtungs-Spritzgusskörper und Fensterbauteil.

Wenn die Innenseite und die Außenseite des Fensterbauteils zueinander parallele Stirnseiten des Fensterbauteils bilden, kann der Ankervorsprung an der umlaufenden Mantelfläche des Fensterbauteils ausgebildet werden. Bevorzugt sind die Innenseite und die Außenseite des Fensterbauteils zueinander parallel, um unerwünschte optische Brechungswirkungen zu vermeiden.

Auf der Außenseite des Beobachtungs-Spritzgusskörpers ist die Anordnung von ausreichend Material des Beobachtungs-Spritzgusskörpers zwischen der Außenfläche des Beobachtungs-Spritzgusskörpers und dem Fensterbauteil bzw. dem Ankervorsprung kein Problem, da an der Außenseite des Messraums keine bündige Außenfläche benötigt wird.

Das Fensterbauteil kann zusammen mit dem Beobachtungs-Spritzgusskörper verwendet werden, ein erstes und ein zweites Erster-Gasbestandteil-Beobachtungs-Wandbauteil zu bilden, wie es oben beschrieben ist**.** Wiederum gilt dann gemäß einer bevorzugten Weiterbildung der Erfindung, dass am Gehäuse das erste und das zweite Erster-Gasbestandteil-Beobachtungs-Wandbauteil zu je einer anderen Seite des Zweiter-Gasbestandteil-Beobachtungs-Wandbauteils angeordnet sind.

Der Ankervorsprung kann auch in Umfangsrichtung mit Abstand voneinander angeordnete Anker-Teilvorsprünge umfassen.

Die vorliegende Erfindung wird nachfolgend anhand der beiliegenden Zeichnungen näher dargestellt. Es stellt dar:
- Fig. 1: eine perspektivische Ansicht einer erfindungsgemäßen Ausführungsform einer Messvorrichtung der vorliegenden Anmeldung,
- Fig. 2: die Messvorrichtung von Fig. 1 in perspektivischer Explosionsansicht,
- Fig. 3: eine Draufsicht auf die Messvorrichtung der Fig. 1 und 2,
- Fig. 4: eine Schnittansicht durch die Messvorrichtung der Fig. 1 bis 3 entlang der Schnittebene A-A von Fig. 3,
- Fig. 5: eine Schnittansicht der Messvorrichtung der Fig. 1 bis 4 entlang der Schnittebene B-B von Fig. 3,
- Fig. 6: eine Ansicht der Messvorrichtung der Fig. 1 bis 5 längs der Strömungsbahn in Richtung eines exspiratorischen Atemgasflusses,
- Fig. 7: eine Schnittansicht durch die Messvorrichtung der Fig. 1 bis 6 längs der Schnittebene C-C von Fig. 6 und
- Fig. 8: eine Schnittansicht eines Beispiels einer Messvorrichtung entsprechend der Ansicht von Figur 5.

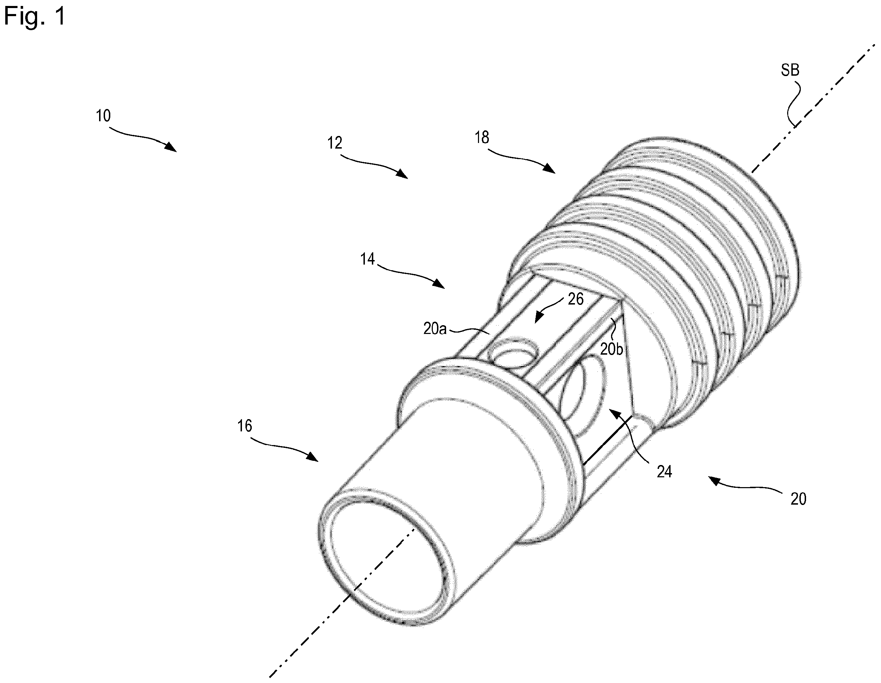

In den Fig. 1 bis 7 ist eine erfindungsgemäße Ausführungsform einer Messvorrichtung allgemein mit 10 bezeichnet. Die Messvorrichtung 10 dient zur Ermittlung wenigstens eines Gasbestandteils eines die Messvorrichtung 10 durchströmenden Gases. Die Messvorrichtung 10 umfasst ein Kunststoffgehäuse 12, welches längs einer im dargestellten Beispiel bevorzugt geradlinigen virtuellen Strömungsbahn SB bidirektional durchströmbar ist.

Das Gehäuse 12 umfasst einen zentralen Beobachtungsbereich 14, eine distale Anschlussformation 16 zum Anschluss einer Gasleitung, insbesondere eines Beatmungsschlauchs, und eine proximale Anschlussformation 18 zum Anschluss einer gasführenden Leitung, insbesondere eines Beatmungsschlauchs oder Beatmungsrohrs.

Die zur Verwendung in einer Beatmungsschlauchanordnung einer Beatmungsvorrichtung zur künstlichen Beatmung von Patienten ausgebildete Messvorrichtung 10 wird üblicherweise mit der distalen Anschlussformation 16 mit der Atemgasquelle der Beatmungsvorrichtung verbunden und wird über die proximale Anschlussformation 18 mit dem zu beatmenden Patienten verbunden.

Das Gehäuse 12 weist einen Rahmenspritzgusskörper 20 und im vorliegenden Beispiel drei Beobachtungs-Wandbauteile 22, 24 und 26 auf, von welchen in Fig. 1 nur die Beobachtungs-Wandbauteile 24 und 26 zu erkennen sind.

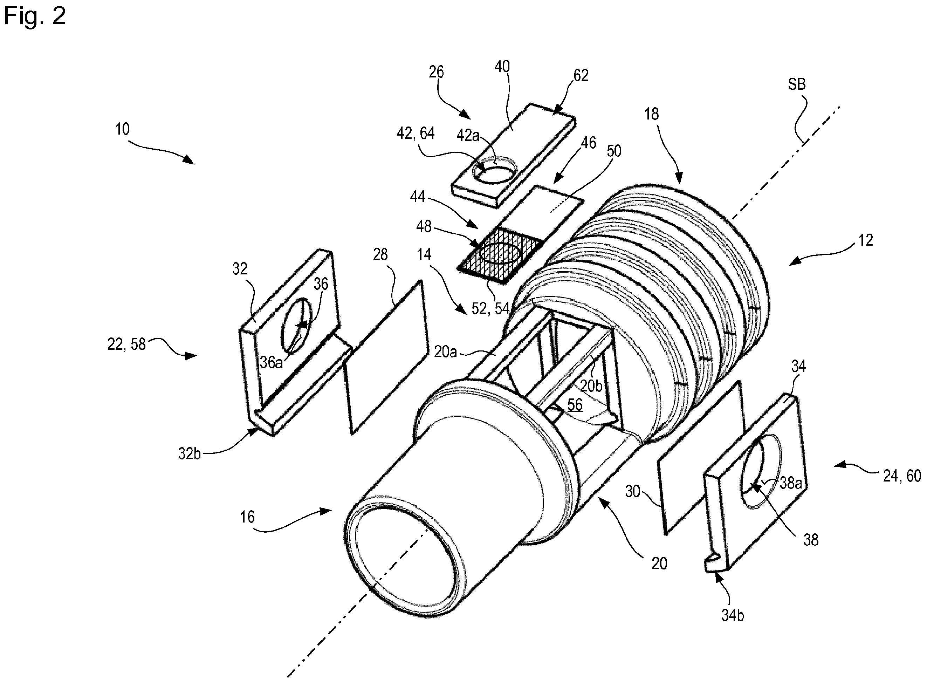

In Fig. 2 ist eine perspektivische Explosionsansicht der Messvorrichtung 10 von Fig. 1 dargestellt.

In Fig. 2 ist auch das in Fig. 1 nicht erkennbare Beobachtungs-Wandbauteil 22 gezeigt. Ebenso ist erkennbar, dass der Rahmen-Spritzgusskörper ein einstückig durch Spritzgießen hergestelltes Bauteil ist, an welchem die Anschlussformationen 16 und 18 ausgebildet sind.

Die beiden Beobachtungs-Wandbauteile 22 und 24 dienen an der Messvorrichtung 10 der Ermittlung von CO₂ als einem ersten Gasbestandteil. Sie sind daher Erster-Gasbestandteil-Beobachtungs-Wandbauteile 22 und 24. Sie sind bevorzugt bezüglich einer zur Strömungsbahn SB orthogonalen Ebene spiegelbildlich ausgebildet, so dass jedes Wandbauteil 22 und 24 auf jeder Seite des Rahmenspritzgusskörpers 20 als ein Erster-Gasbestandteil-Beobachtungs-Wandbauteil der Messvorrichtung 10 einsetzbar sind. Jeder der beiden Erster-Gasbestandteil-Beobachtungs-Wandbauteile 22 und 24 weist einen Folienkörper 28 bzw. 30 auf, welcher aus BOPP gebildet ist oder wenigstens auf seiner von der Strömungsbahn SB weg weisenden Seite eine Lage aus BOPP aufweist.

Beispielhaft und nicht maßstäblich ist der Folienkörper 28 im Detail gezeigt. Im dargestellten Beispiel umfasst der Folienkörper 28, von außen nach innen, eine Schutzfolie 28a vorzugsweise aus Polycarbonat, eine Haftvermittlungslage 28b, vorzugsweise aus Reinacrylat-Klebstoff, und eine BOPP-Folienlage 28c. Der Folienkörper 30 kann identisch zum Folienkörper 28 aufgebaut sein. Abweichend von der Darstellung kann der Folienkörper 28 nur eine Folienlage aufweisen. Dann kann der Folienkörper 28 identisch zum beispielhaft dargestellten Folienkörper 30 aufgebaut sein.

An die Polycarbonat-Lage 28a des Folienkörpers 28 ist ein Beobachtungs-Spritzgusskörper 32 angespritzt. An die BOPP-Lage des Folienkörpers 30 ist ein Beobachtungs-Spritzgusskörper 34 angespritzt. Hierzu werden die Folienkörper 28 und 30 in eine jeweilige Spritzgusskavität eingelegt und dann die Beobachtungs-Spritzgusskörper 32 und 34 an die Folienkörper 28 bzw. 30 in einem Spritzgussvorgang angespritzt.

Jeder der Beobachtungs-Spritzgusskörper 32 und 34 weist eine sie in Dickenrichtung durchsetzende Aussparung 36 bzw. 38 auf, welche an ihrem der virtuellen Strömungsbahn SB näher gelegenen Ende von dem jeweils zugeordneten Folienkörper 28 bzw. 30 bedeckt ist.

Eine Randfläche 36a der Beobachtungs-Aussparung 36 und eine Randfläche 38a der Beobachtungs-Aussparung 38 sind jeweils als sich, vorzugsweise konisch, zum Folienkörper 28 bzw. 30 hin verjüngende Randflächen ausgebildet, so dass jede Randfläche 36a und 38a jeweils als Zentrierfläche für die Anordnung des Wandbauteils 22 bzw. 24 in einer Spritzgusskavität zur Herstellung des Rahmen-Spritzgusskörpers 20 dienen kann. Das Wandbauteil 22 kann dann anhand seiner Randfläche 36a zentriert werden und das Wandbauteil 24 anhand seiner Randfläche 38a. Hierdurch ist eine sehr exakte Anordnung der Wandbauteile 22 und 24 in der Spritzgusskavität zur Herstellung des Rahmen-Spritzgusskörpers 20 möglich.

Wegen der bevorzugten spiegelsymmetrischen Ausbildung zu einer zur virtuellen Strömungsbahn SB orthogonalen Ebene befinden sich die Aussparungen 36 und 38 in der dargestellten Ausführungsform bezüglich der virtuellen Strömungsbahn SB in der Längsmitte der Beobachtungs-Spritzgusskörper 32 bzw. 34.

Die Beobachtungs-Spritzgusskörper 32 und 34 weisen zur Erleichterung ihrer Anordnung am Rahmen-Spritzgusskörper 20 und insbesondere zur Anordnung in der Spritzgusskavität zur Herstellung des Rahmen-Spritzgusskörpers 20 an ihrem in den Fig. 1 und 2 jeweiligen unteren Ende je eine Ausrichtformation 32b bzw. 34b auf.

Wenngleich die Fig. 2 eine Explosionsdarstellung der Messvorrichtung 10 zeigt, sollte verstanden werden, dass aufgrund des Anspritzens des Rahmen-Spritzgusskörpers 20 an die Beobachtungs-Wandbauteile 22, 24 und 26 die so gebildete Messvorrichtung 10 nicht mehr zerlegbar ist, sondern dass der Rahmen-Spritzgusskörper 20 und die Beobachtungs-Wandbauteile 22, 24 und 26 eine im Wesentlichen stoffschlüssige Einheit bilden.

Die Erster-Gasbestandteil-Beobachtungs-Wandbauteile 22 und 24 weisen jeweils sowohl auf ihrer im fertig montierten Zustand zur virtuellen Strömungsbahn SB hin weisenden Seite, an welcher sich die jeweiligen Folienkörper 28 bzw. 30 befinden, sowie auf ihrer von der virtuellen Strömungsbahn SB weg weisenden Außenseite, die gebildet ist durch eine Außenseite der Beobachtungs-Spritzgusskörper 32 bzw. 34, je eine ebene Außenfläche auf.

Das Wandbauteil 26 dient in der dargestellten Ausführungsform der Ermittlung eines O₂-Anteils in dem die Messvorrichtung 10 durchströmenden Gas. Das Wandbauteil 26 ist daher in Abgrenzung zu den Erster-Gasbestandteil-Beobachtungs-Wandbauteilen 22 und 24 ein Zweiter-Gasbestandteil-Beobachtungs-Wandbauteil 26 im Sinne der vorliegenden Anmeldung und weist einen Beobachtungs-Spritzgusskörper 40 auf, welcher in Dickenrichtung durch eine Aussparung 42 durchsetzt ist.

Der Beobachtungs-Spritzgusskörper 40 ist wie die anderen beiden Beobachtungs-Spritzgusskörper 32 und 34 an einen Folienkörper, hier der Folienkörper 44 spritzgusstechnisch angespritzt.

Im Gegensatz zu den Folienkörpern 28 und 30, welche lediglich eine einzige BOPP-Lage umfassen können, ist der Folienkörper 44 mehrlagig. Auch die Folienkörper 28 und 30 können jedoch mehrlagig ausgebildet sein.

Die dem Beobachtungs-Spritzgusskörper 40 nächstgelegene Folienlage des Folienkörpers 44 ist eine BOPP-Folienlage 46. Die BOPP-Folienlage 46 weist dort eine sie durchsetzende Aussparung 48 auf, wo nach dem Anspritzen des Beobachtungs-Spritzgusskörpers 40 dessen Aussparung 42 gelegen ist.

Auf der von dem Beobachtungs-Spritzgusskörper 40 wegweisenden Seite der BOPP-Folie 46 befindet sich in einem der proximalen Anschlussformation 18 näher gelegenen Bereich der BOPP-Folie 46 eine luminophorhaltige Folie 50. Das Luminophor in der luminophorhaltigen Folielage 50 kann durch den vorzugsweise transparenten Beobachtungs-Spritzgusskörper 40 hindurch zum Abstrahlen von elektromagnetischer Strahlung, insbesondere von Licht, angeregt werden und kann durch den Beobachtungs-Spritzgusskörper 40 hindurch in seinem angeregten Abstrahlverhalten beobachtet werden.

Sauerstoff in dem die Messvorrichtung 10 entlang der virtuellen Strömungsbahn SB durchströmenden Gas kommt mit dem angeregten Luminophor der Folienlage 50 in Kontakt, wodurch das Luminophor abgelöscht, also "entregt", wird und somit sein Abstrahlverhalten abhängig von der Sauerstoffkonzentration in dem die Messvorrichtung 10 durchströmenden Gas verändert.

In einem näher bei der distalen Anschlussformation 16 gelegenen Abschnitt des Folienkörpers 44 weist dieser auf einer von dem Beobachtungs-Spritzgusskörper 40 wegweisenden Seite der BOPP-Folienlage 46 eine Metallfolie 52 auf, welche auf ihrer zur BOPP-Folienlage 46 und somit zum Beobachtungs-Spritzgusskörper 40 hinweisenden Seite mit schwarzem Lack 54 beschichtet ist.

Die mit schwarzem Lack 54 beschichtete Metallfolie 52 bildet somit eine Temperaturmess-Folienlage, die durch die Aussparung 42 hindurch beobachtbar ist. Die Metallfolie 52, bevorzugt eine Aluminiumfolie 52, mit ebenso bevorzugt einer Materialdicke im einstelligen µm-Bereich, nimmt aufgrund ihrer guten Wärmeleitfähigkeit die Temperatur des die Messvorrichtung 10 längs der virtuellen Strömungsbahn SB durchströmenden Gases an. Der schwarze Lack 54 strahlt eine für die Temperatur der Metallfolie 52 charakteristische Wärmestrahlung durch die Aussparung 42 nach außen ab, wo diese als Infrarotstrahlung beobachtbar ist. So kann die Temperatur des die Messvorrichtung 10 durchströmenden Gases ermittelt werden. Das Wandbauteil 26 ist daher auch ein Temperatur-Beobachtungs-Wandbauteil 26.

Durch die Aussparungen 36 und 38 kann die Messvorrichtung 10, wiederum genauer ein von den Beobachtungs-Wandbauteilen 22, 24 und 26 umgebener Messraum 56 im Inneren der Messvorrichtung 10, mit Infrarotstrahlen durchstrahlt werden. Hierzu wird mit einer äußeren Infrarot-Strahlungsquelle Infrarotstrahlung durch eine der Aussparungen 36 oder 38 in den Messraum 56 derart eingestrahlt, dass die Infrarotstrahlung durch die jeweils andere Aussparung beobachtbar ist. Das zur Messung des Kohlendioxidgehalts des Atemgases verwendbare infrarotspektroskopische Verfahren ist hinlänglich bekannt.

Da die beiden Beobachtungs-Wandbauteile 22 und 24 im Wesentlichen identisch aufgebaut sind, kann jedes der beiden Wandbauteile 22 und 24 zum Einstrahlen von Infrarotstrahlung in den Messraum 56 und das jeweils andere zum Beobachten der aus dem Messraum 56 nach Durchgang durch diesen austretenden Infrarotstrahlung verwendet werden.

Daher bildet das erste Erster-Gasbestandteil-Beobachtungs-Wandbauteil 22 einen Beobachtungsabschnitt 58 und das zweite Erster-Gasbestandteil-Beobachtungs-Wandbauteil 24 einen Beobachtungsabschnitt 60.

Das Beobachtungs-Wandbauteil 26 umfasst dagegen zwei gesonderte Beobachtungsabschnitte, nämlich einen Beobachtungsabschnitt 62 zur Beobachtung des Strahlungsverhaltens des Luminophors der luminophorhaltigen Folienlage 50 und einen Temperatur-Beobachtungsabschnitt 64 im Bereich der Ausnehmung 42 zur Beobachtung der von der Temperaturmess-Folienlage 52 mit Lackauftrag 54 ausgehenden Wärmestrahlung zur Feststellung der Temperatur des die Messvorrichtung 10 durchströmenden Gases. Es kann auch nur einer der Beobachtungsabschnitte 62 und 64 vorgesehen sein.

Auch die Randfläche 42a der Ausnehmung 42 im Beobachtungs-Spritzgusskörper 26 ist im Übrigen von außen zur Folienanordnung 44 hin konisch verjüngt ausgebildet, um das sowohl zur Temperaturermittlung als auch zur Ermittlung des Sauerstoffgehalts verwendbare Beobachtungs-Wandbauteil 26 exakt positioniert und zentriert in der Spritzgusskavität zur Herstellung des Rahmen-Spritzgusskörpers 20 anordnen zu können.

Der Beobachtungs-Spritzgusskörper 40 des Beobachtungs-Wandbauteils 26 ist auf seiner zum Messraum 56 hin weisenden Seite sowie auf seiner vom Messraum 56 weg weisenden Außenseite jeweils durch eine ebene Fläche begrenzt. Vorzugsweise sind die beiden ebenen Grenzflächen zueinander parallel. Dies gilt auch für die zuvor genannten Beobachtungs-Spritzgusskörper 32, 34. Wegen unterschiedlicher Schichtdicken der mit der schwarzen Lackschicht 54 versehenen Metallfolie 52 einerseits und der luminophorhaltigen Folienlage 50 andererseits kann die zum Messraum 56 hinweisende Grenzfläche des Beobachtungs-Wandbauteils 26 zwei, vorzugsweise ebene, Flächenabschnitte aufweisen, welche zueinander in einer zur Strömungsbahn SB orthogonalen Richtung versetzt sind.

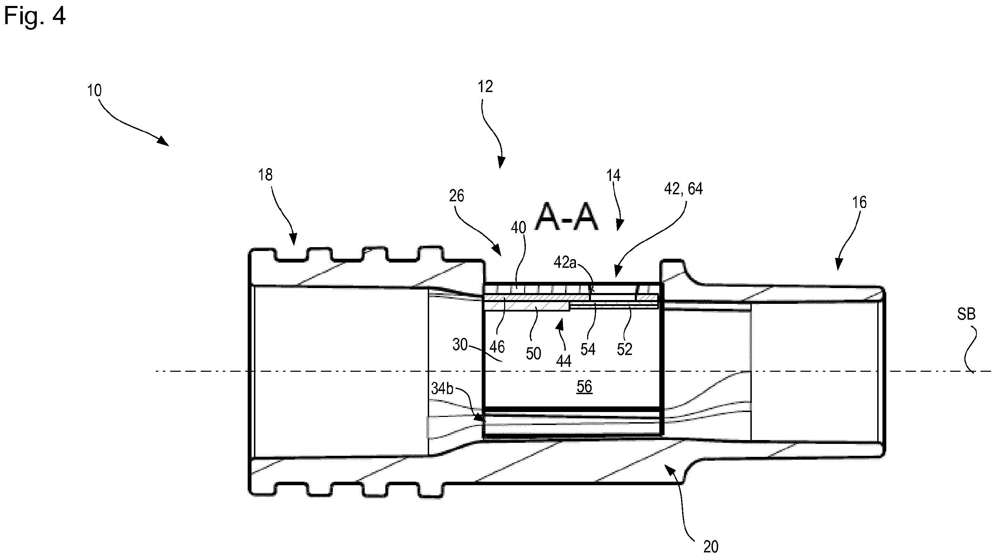

In der Schnittansicht von Fig. 4, bei welcher der Betrachter durch den Messraum 56 hindurch auf die BOPP-Folienlage 30 blickt, ist der Folienkörper 44 des Beobachtungs-Wandbauteils 26 in Draufsicht zu erkennen.

Dabei ist insbesondere zu erkennen, dass der Folienkörper 44 keine einheitliche Dicke aufweisen muss. Beispielsweise kann dieser im Bereich der luminophorhaltigen Folienlage 50 dicker ausgebildet sein als im Bereich der Temperaturmess-Folienlage 52 mit der schwarzen Beschichtung 54 darauf.

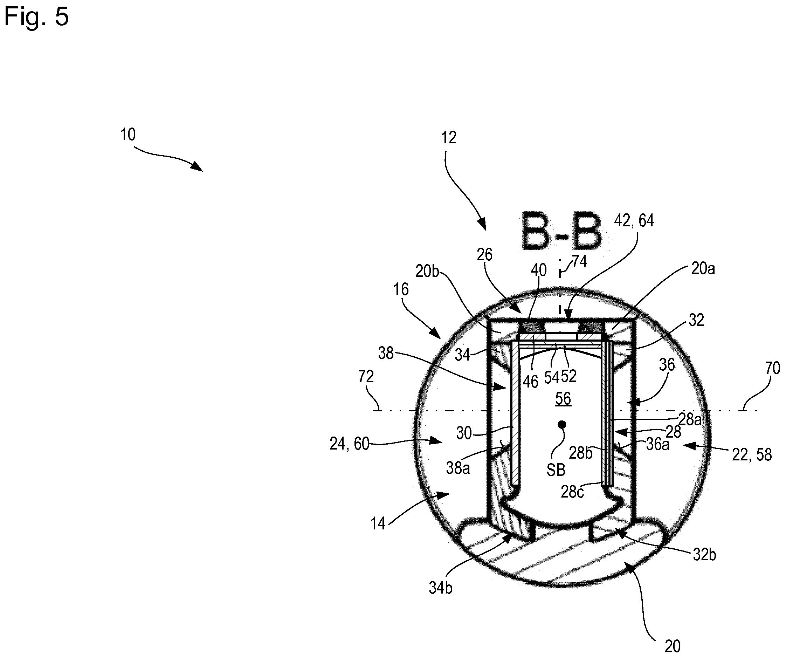

Zu erkennen ist in Fig. 5, wieviel Flächen der Beobachtungs-Wandteile 22, 24 und 26 beim spritzgusstechnischen Erzeugen des Rahmen-Spritzgussköpers 20 von Flächen des Rahmen-Spritzgusskörpers 20 benetzt werden, so dass eine stoffschlüssige Verbindung zwischen den bevorzugt aus wenigstens kompatiblen, vorzugsweise gleichen, Kunststoffen hergestellten Beobachtungs-Spritzgusskörpern 32, 34 und 40 einerseits und dem Rahmen-Spritzgusskörper 20 andererseits hergestellt wird. Insbesondere die Anordnungsformationen 32b und 34b der Beobachtungs-Spritzgusskörper 32 bzw. 34 weisen eine große vom Rahmen-Spritzgusskörper 20 benetzte Fläche auf, die sogar abgewinkelt ist und eine Außenfläche der Anordnungsformationen 32b und 34b umgreift.

In Fig. 5 dargestellt sind die virtuellen Aussparungsachsen 70, 72 und 74 der Aussparungen 36, 38 bzw. 42. Die Aussparungen 36, 38 und 42 verjüngen sich längs der sie zentral durchsetzenden virtuellen Aussparungsachsen 70, 72 bzw. 74 von außerhalb des Messraums 56 in Richtung zum Messraum 56 hin. Die Aussparungsachsen 70, 72 und 74 können Konusachsen der konischen Randflächen 36a, 38a und 42a der jeweiligen Aussparungen 36, 38 bzw. 42 sein, etwa wenn die jeweiligen Randflächen rotationssymmetrisch ausgebildet sind.

Da die beiden Beobachtungsabschnitte 58 und 60 zur Durchleuchtung des zwischen ihnen angeordneten Messraums 56 ausgebildet sind, sind die zugehörigen Aussparungsachsen 70 und 72 bevorzugt kollinear.

Wie Fig. 5 außerdem zeigt, befindet sich das Zweiter-Gasbestandteil-Wandbauteil 26 zwischen den beiden Erster-Gasbestandteil-Beobachtungs-Wandbauteilen 22 und 24, oder anders ausgedrückt: Die Erster-Gasbestandteil-Beobachtungs-Wandbauteile 22 und 24 befinden sich jeweils auf unterschiedlichen Seiten des Zweiter-Gasbestandteil-Beobachtungs-Wandbauteils 26. Vorzugsweise überlappen sich die Erstreckungsbereiche der Wandbauteile 22, 24 und 26 längs der virtuellen Strömungsbahn SB, so dass der Messraum 56 längs der Strömungsbahn SB kurz ausgebildet werden kann.

In Fig. 5 ist weiter zu erkennen, wie die vom Messraum 56 weg weisenden Außenflächen der Beobachtungs-Spritzgussbauteile 32, 34 und 40 eben ausgebildet sind und zueinander parallel bzw. in einem rechten Winkel angeordnet sind. Die zwischen jedem der Erster-Gasbestandteil-Beobachtungs-Wandbauteile 22 bzw. 24 einerseits und dem Zweiter-Gasbestandteil-Beobachtungs-Wandbauteil 26 andererseits angeordneten, parallel zur Strömungsbahn SB verlaufenden Stege 20a und 20b des Rahmen-Spritzgusskörpers 20 im Bereich des Messraums 56 schließen mit ihren Außenflächen bündig an die Außenflächen der Wandbauteile 22, 24 und 26 an, so dass eine Messtechnikvorrichtung orthogonal zur virtuellen Strömungsbahn SB von der Seite des Wandbauteils 26 kommend im Beobachtungsbereich 14 der Messvorrichtung 10 auf diese aufgesteckt werden kann. Eine solche Messvorrichtung kann eine Infrarotstrahlungsquelle und einen dieser Quelle in Richtung orthogonal zur virtuellen Strömungsbahn SB gegenüberliegenden Infrarotsensor aufweisen. Weiter kann die Messvorrichtung zwischen der Infrarotstrahlungsquelle und dem Infrarotsensor eine Anregungsstrahlungsquelle zur Anregung des Luminophors in der luminophorhaltigen Folienlage 50 und eine Erfassungsvorrichtung zur Beobachtung des Ablöschverhaltens desselben aufweisen. Ebenso kann darin ein weiterer Infrarotsensor zur Erfassung der Wärmestrahlung von der Temperaturmess-Folienlage 52 angeordnet sein. Eine solche Messvorrichtung kann einfach auf den Beobachtungsbereich 14 der Messvorrichtung 10 aufgesteckt und wieder von diesem abgezogen werden.

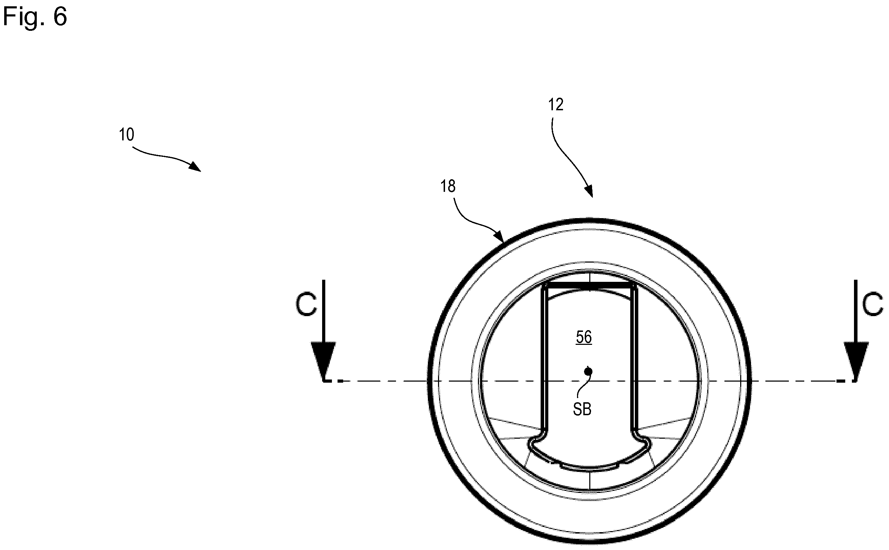

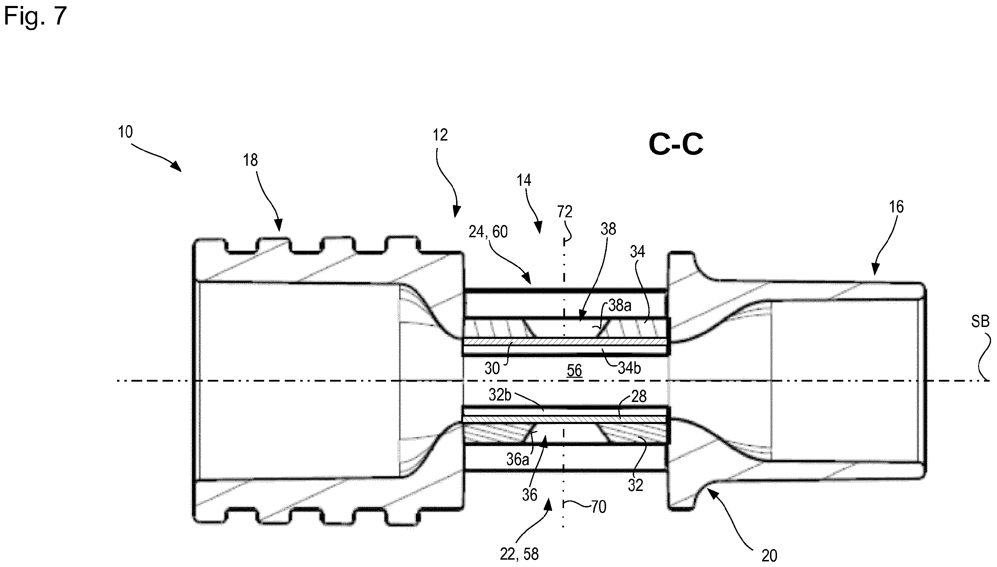

Fig. 7 zeigt eine Schnittansicht durch die Messvorrichtung 10 entlang der Schnittebene C-C von Fig. 6. Die beiden Folienkörper 28 und 30 sind parallel zur virtuellen Strömungsbahn SB und parallel zueinander ausgerichtet.

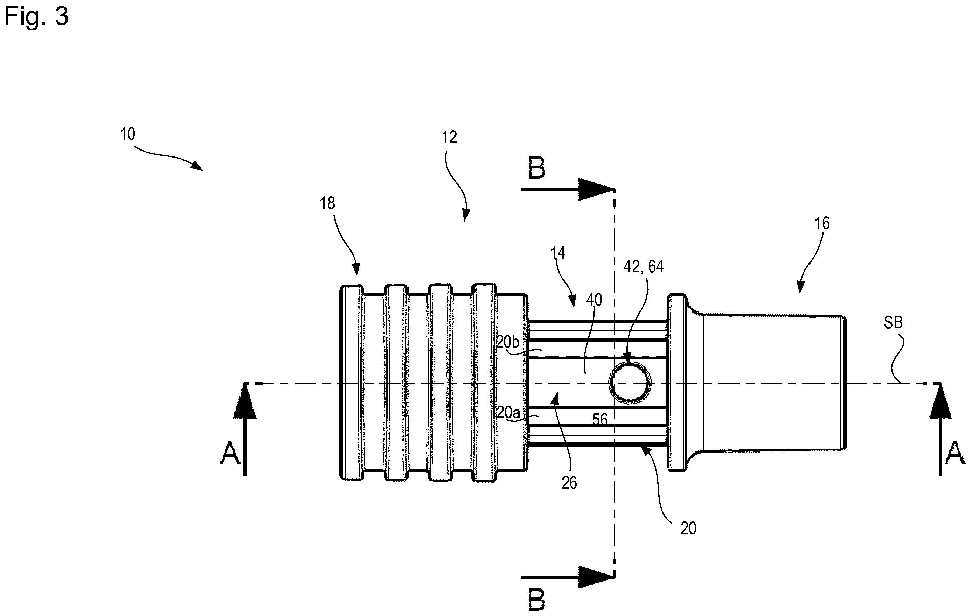

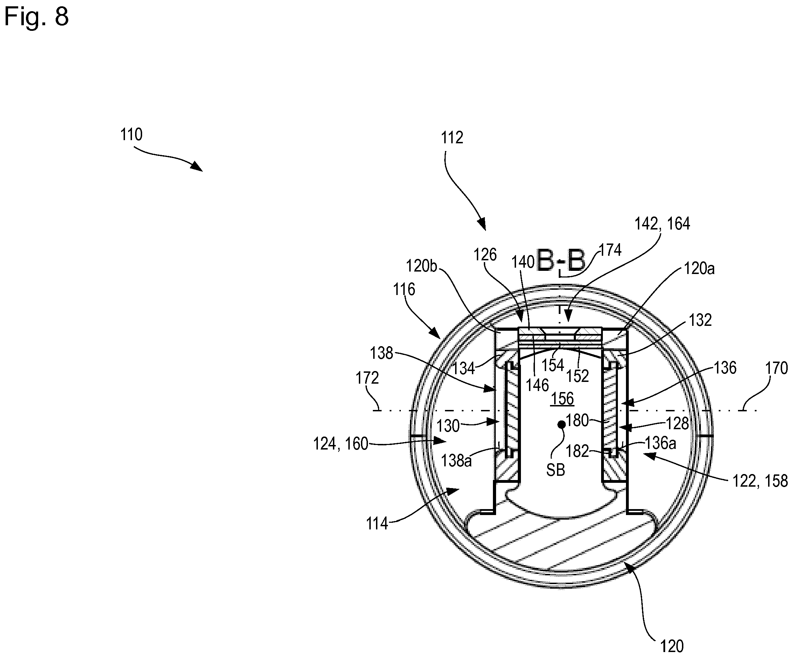

Fig. 8 zeigt eine Schnittansicht einer zweiten Ausführungsform einer Messvorrichtung 110 in der Schnittebene B-B von Figur 3, jedoch in entgegengesetzter Blickrichtung auf die Schnittebene B-B, wie die in Figur 3 angegebene. Die Ansicht von Figur 8 entspricht in der Schnittebene grundsätzlich der Darstellung von Figur 5. Gleiche bzw. funktionsgleiche Bauteile und Bauteilabschnitte wie in der ersten Ausführungsform der Figuren 1 bis 7 sind in Figur 8 mit den gleichen Bezugszeichen versehen, jedoch erhöht um die Zahl 100.

Das Beispiel von Figur 8 wird nachfolgend nur insoweit beschrieben werden, als es sich von der ersten Ausführungsform der Figuren 1 bis 7 unterscheidet, deren Beschreibung ansonsten auch für die Figur 8 gilt.

Ein eher unbedeutender Unterschied liegt darin, dass die Beobachtungs-Spritzgusskörper 132 und 134 an ihren vom Beobachtungs-Wandbauteil 126 fernliegenden Endbereichen keine Ausrichtformationen aufweisen.

In den Beobachtungs-Aussparungen 136 und 138 befinden sich anstelle von Folienkörpern Fensterbauteile 128 bzw. 130 aus Glas. Die Fensterbauteile 128 und 130 sind identisch ausgebildet und lediglich relativ zueinander bezüglich einer durch die Strömungsbahn SB und die Aussparungsachse 174 aufgespannten Spiegelsymmetrieebene spiegelverkehrt in der Messvorrichtung 110 angeordnet. Die Fensterbauteile 128 und 130 weisen im dargestellten Beispiel einen kreisrunden Umfang auf. Dies muss jedoch nicht so sein. Die Fensterbauteile 128 und 130 können alternativ einen ovalen oder einen polygonalen Umfang aufweisen.

Aufgrund ihrer identischen Ausbildung reicht es aus, nachfolgend das Fensterbauteil 128 näher zu beschreiben. Dessen Beschreibung gilt unter Beachtung der oben genannten Spiegelsymmetrie auch für das Fensterbauteil 130.

Das Fensterbauteil 128 weist einen zentralen Fensterabschnitt 180 auf, welcher im dargestellten Beispiel eine im Wesentlichen zylindrische Gestalt aufweist, wobei die Aussparungsachse 170 vorzugsweise die Zylinderachse des Fensterabschnitts 180 ist.

Vollständig um den Fensterabschnitt 180 umlaufend weist das Fensterbauteil 128 einen radial vom Fensterabschnitt 180 auskragenden Ankervorsprung 182 auf, welcher eine geringere Dicke aufweist als der Fensterabschnitt 180. Der Ankervorsprung 182 ist längs seines gesamten Umlaufs um die Aussparungsachse 170 an drei Seiten U-förmig von Material des Beobachtungs-Spritzgusskörpers 132 umgeben. Das Fensterbauteil 180 wurde mit seinem Ankervorsprung 182 bei der spritzgusstechnischen Herstellung des Beobachtungs-Spritzgusskörpers 132 von diesem umspritzt. Hierdurch wurde eine sowohl mechanisch feste als auch gasdichte Verbindung des Fensterbauteils 128 mit dem Beobachtungs-Spritzgusskörper 132 erreicht.

Die Fensterbauteile 128 und 130 sind vorliegend aus Saphirglas oder aus Chalkogenidglas hergestellt, d. h. sie bestehen entweder aus Aluminiumoxid oder aus Germanium.

Die Innenfläche des Fensterabschnitts 180 des Fensterbauteils 128 ist bündig mit der Innenfläche des Beobachtungs-Spritzgusskörpers 132 angeordnet. Hierdurch werden Stufen an den den Messraum 156 begrenzenden Innenwänden vermieden, an denen sich ansonsten Feuchtigkeit unerwünschterweise niederschlagen könnte.

Der Ankervorsprung 182 bildet zusammen mit dem Fensterabschnitt 180 eine ebene Außenfläche des Fensterbauteils 128.

## Patentansprüche

1. Messvorrichtung (10) zur Ermittlung wenigstens eines Gasbestandteils eines in einem Messraum (56) der Messvorrichtung (10) vorhandenen Gases, wobei die Messvorrichtung (10) ein den Messraum (56) umgebendes Gehäuse (12) umfasst, von welchem wenigstens ein Gehäusewandabschnitt als Beobachtungsabschnitt (58, 60, 62) zur Erfassung von vom Beobachtungsabschnitt (58, 60, 62, 64) in Richtung vom Messraum (56) weg ausgehender elektromagnetischer Strahlung ausgebildet ist, wobei der Beobachtungsabschnitt (58, 60, 62, 64) wenigstens eine Folienlage (28a, 28c, 46, 50, 52) umfasst und wobei das Gehäuse (12) als Kunststoff-Spritzgussgehäuse ausgebildet ist, **dadurch gekennzeichnet, dass** der Beobachtungsabschnitt (58, 60, 62, 64) wenigstens ein Beobachtungs-Wandbauteil (22, 24, 26), umfassend einen an die wenigstens eine Folienlage (28a, 28c, 46, 50, 52) angespritzten Beobachtungs-Spritzgusskörper (32, 34, 40), aufweist, wobei das Gehäuse (12) das wenigstens eine Beobachtungs-Wandbauteil (58, 60, 62) und einen an das Beobachtungs-Wandbauteil (22, 24, 26) angespritzten Rahmen-Spritzgusskörper (20) umfasst.

2. Messvorrichtung (10) nach Anspruch 1,
**dadurch gekennzeichnet, dass** der Messraum (56) längs einer virtuellen Strömungsbahn (SB) von Gas durchströmbar ist.

3. Messvorrichtung (10) nach Anspruch 1 oder 2,
**dadurch gekennzeichnet, dass** der Beobachtungs-Spritzgusskörper (32, 34, 40) eine Beobachtungs-Aussparung (36, 38, 42) aufweist, innerhalb welcher die wenigstens eine Folienlage (28a, 28c46, 50, 52) zugänglich ist.

4. Messvorrichtung (10) nach Anspruch 3,
**dadurch gekennzeichnet, dass** eine Randfläche (36a, 38a, 42a) der Beobachtungs-Aussparung (36, 38, 42) sich in Richtung zur wenigstens einen Folienlage (28a, 28c, 46, 50, 52) hin verjüngt und so als Zentrierfläche für einen Werkzeugangriff ausgebildet ist.

5. Messvorrichtung (10) nach einem der vorhergehenden Ansprüche,
**dadurch gekennzeichnet, dass** die wenigstens eine Folienlage (28a, 28c 46) für elektromagnetische Strahlung im infraroten Spektralbereich durchlässig ist.

6. Messvorrichtung (10) nach Anspruch 5,
**dadurch gekennzeichnet, dass** die wenigstens eine Folienlage (28a, 28c, 46, 50, 52) eine biaxial orientierte Polymerfolie (28c, 46) umfasst.

7. Messvorrichtung (10) nach einem der vorhergehenden Ansprüche,
**dadurch gekennzeichnet, dass** sie ein erstes und ein zweites Erster-Gasbestandteil-Beobachtungs-Wandbauteil (22, 24) umfasst, welche zur Erfassung eines ersten Gasbestandteils derart am Gehäuse (12) vorgesehen sind, dass sich wenigstens ein Abschnitt des Messraums (56) zwischen ihnen befindet, wobei das erste und das zweite Erster-Gasbestandteil-Beobachtungs-Wandbauteil (22, 24) derart am Gehäuse (12) angeordnet sind, dass elektromagnetische Strahlung, welche durch einen Beobachtungsabschnitt (58 oder 60) eines der beiden Erster-Gasbestandteil-Beobachtungs-Wandbauteile (22, 24) in den Messraum (56) eingestrahlt wird, durch den Beobachtungsabschnitt (60 oder 58) des jeweils anderen Erster-Gasbestandteil-Beobachtungs-Wandbauteils (22, 24) aus dem Messraum (56) ausstrahlen kann.

8. Messvorrichtung (10) nach einem der vorhergehenden Ansprüche,
**dadurch gekennzeichnet, dass** die wenigstens eine Folienlage (28a, 28c, 46, 50, 52) als Temperaturmess-Folienlage (52) eine Metallfolie (52) umfasst.

9. Messvorrichtung (10) nach einem der vorhergehenden Ansprüche,
**dadurch gekennzeichnet, dass** die wenigstens eine Folienlage (28a, 28c, 46, 50, 52) als Zweiter-Gasbestandteil-Folienlage (50) zur Erfassung eines vom ersten verschiedenen zweiten Gasbestandteils eine Fotolumineszenz-Lage (50) mit wenigstens einem darin aufgenommenem Luminophor aufweist.

10. Messvorrichtung (10) nach den Ansprüchen 8 und 9,
**dadurch gekennzeichnet, dass** sie ein Zweiter-Gasbestandteil-Beobachtungs-Wandbauteil (26) mit der Zweiter-Gasbestandteil-Folienlage (50) aufweist, welches auch die Temperaturmess-Folienlage (52) aufweist.

11. Messvorrichtung (10) nach einem der Ansprüche 9 oder 10,
**dadurch gekennzeichnet, dass** die Zweiter-Gasbestandteil-Folienlage (50) auf ihrer vom Messraum (56) abgewandten Seite vollständig vom Beobachtungs-Spritzgusskörper (40) bedeckt ist, wobei der Beobachtungs-Spritzgusskörper (40) für elektromagnetische Strahlung im Wellenlängenbereich der von der Fotolumineszenz-Lage (50) abgestrahlten Fotolumineszenz-Strahlung durchlässig ist.

12. Messvorrichtung (10) nach einem der vorhergehenden Ansprüche, unter Einbeziehung der Ansprüche 7 und 9,
**dadurch gekennzeichnet, dass** sie sowohl das erste und das zweite Erster-Gasbestandteil-Beobachtungs-Wandbauteil (22, 24) als auch ein Zweiter-Gasbestandteil-Beobachtungs-Wandbauteil (26) mit der Zweiter-Gasbestandteil-Folienlage (50) aufweist, wobei am Gehäuse (10) das erste und das zweite Erster-Gasbestandteil-Beobachtungs-Wandbauteil (22, 24) zu je einer anderen Seite des Zweiter-Gasbestandteil-Beobachtungs-Wandbauteils (26) angeordnet sind.

13. Messvorrichtung (10) nach einem der vorhergehenden Ansprüche,
**dadurch gekennzeichnet, dass** an wenigstens einem Längsende des Gehäuses eine Anschlussformation (16, 18) angeordnet ist, welche zum Anschluss einer Schlauch- oder/und Rohrleitung ausgebildet ist.

14. Verfahren zur Herstellung eines Beobachtungsabschnitts (58, 60, 62, 64) einer Messvorrichtung (10) zur Ermittlung eines Gasbestandteils eines in einem Messraum (56) der Messvorrichtung (10) vorhandenen Gases, wobei der Messraum (56) wenigstens teilweise von dem Beobachtungsabschnitt (58, 60, 62, 64) eingefasst ist, das Verfahren umfassend die folgenden Schritte:
- Einlegen eines Folienkörpers (28, 30, 46, 50, 52) mit wenigstens einer Folienlage (28a, 28c, 46, 50, 52) in eine Spritzgusskavität,
- Anspritzen eines Beobachtungs-Spritzgusskörpers (32, 34, 40) an den Folienkörper (28, 30, 46, 50, 52, 54) und dadurch Bilden eines Beobachtungs-Wandbauteils (22, 24, 26), und
- Anspritzen eines zweiten Spritzguss-Kunststoffkörpers (20) an das Beobachtungs-Wandbauteil (22, 24, 26) und dadurch Herstellen des Beobachtungsabschnitts (58, 60, 62, 64).

## Claims

1. Measuring device (10) for measuring at least one gas constituent of a gas present in a measuring chamber (56) of the measuring device (10), where the measuring device (10) comprises a housing (12) surrounding the measuring chamber (56), of which at least one housing wall section is configured as an observation section (58, 60, 62) for the acquisition of electromagnetic radiation emitted from the observation section (58, 60, 62, 64) in a direction away from the measuring chamber (56), where the observation section (58, 60, 62, 64) comprises at least one foil layer (28a, 28c, 46, 50, 52) and where the housing (12) is configured as a synthetic material injection molded housing,
**characterized in that** the observation section (58, 60, 62, 64) exhibits at least one observation wall component (22, 24, 26), comprising an observation injection-molded body (32, 34, 40) injected onto the at least one foil layer (28a, 28c, 46, 50, 52), where the housing (12) comprises the at least one observation wall component (58, 60, 62) and a frame injection-molded body (20) injected onto the observation wall component (22, 24, 26).

2. Measuring device (10) according to Claim 1,
**characterized in that** the measuring chamber (56) allows the flowthrough of gas along a virtual flow path (SB).

3. Measuring device (10) according to Claim 1 or 2,
**characterized in that** the observation injection-molded body (32, 34, 40) exhibits an observation cutout (36, 38, 42), within which the at least one foil layer (28a, 28c, 46, 50, 52) is accessible.

4. Measuring device (10) according to Claim 3,
**characterized in that** a lateral face (36a, 38a, 42a) of the observation cutout (36, 38, 42) tapers in a direction towards at least one foil layer (28a, 28c, 46, 50, 52) and thus is configured as a centering surface for tool engagement.

5. Measuring device (10) according to one of the preceding claims,
**characterized in that** the at least one foil layer (28a, 28c, 46) is transparent to electromagnetic radiation in the infrared spectral range.

6. Measuring device (10) according to Claim 5,
**characterized in that** the at least one foil layer (28a, 28c, 46, 50, 52) comprises a biaxially oriented polymer foil (28c, 46).

7. Measuring device (10) according to one of the preceding claims,
**characterized in that** it comprises a first and a second first gas constituent observation wall component (22, 24), which are provided for capturing a first gas constituent at the housing (12) in such a way that at least one section of the measuring chamber (56) is located between them, where the first and the second first gas constituent observation wall component (22, 24) are arranged in such a way at the housing (12) that electromagnetic radiation which is radiated into the measuring chamber (56) through an observation section (58 or 60) of one of the two first gas constituent observation wall components (22, 24), can radiate out of the measuring chamber (56) through the observation section (60 or 58) of the respectively other first gas constituent observation wall component (22, 24).

8. Measuring device (10) according to one of the preceding claims,
**characterized in that** the at least one foil layer (28a, 28c, 46, 50, 52) comprises as a temperature measurement foil layer (52) a metal foil (52).

9. Measuring device (10) according to one of the preceding claims,
**characterized in that** the at least one foil layer (28a, 28c, 46, 50, 52) exhibits as second gas constituent foil layer (50) for capturing a second gas constituent differing from the first one a photoluminescent layer (50) with at least one luminophore accommodated in it.

10. Measuring device (10) according to one of the claims 8 and 9,
**characterized in that** it exhibits a second gas constituent observation wall component (26) with the second gas constituent foil layer (50), which also exhibits the temperature measurement foil layer (52).

11. Measuring device (10) according to one of the claims 9 or 10,
**characterized in that** the second gas constituent foil layer (50) is covered completely by the observation injection-molded body (40) on its side facing away from the measuring chamber (56), where the observation injection-molded body (40) is transparent to electromagnetic radiation in the wavelength range of the photoluminescent radiation emitted from the photoluminescent layer (50).

12. Measuring device (10) according to one of the preceding claims, having regard to Claims 7 and 9,
**characterized in that** it exhibits both the first and the second first gas constituent observation wall component (22, 24) and a second gas constituent observation wall component (26) with the second gas constituent foil layer (50), where at the housing (10) the first and the second first gas constituent observation wall component (22, 24) are each arranged at a different side of the second gas constituent observation wall component (26).

13. Measuring device (10) according to one of the preceding claims,
**characterized in that** at least at one longitudinal end of the housing, a connector formation (16, 18) is arranged at each which is configured for connecting a hose and/or pipe line.

14. Method for fabricating an observation section (58, 60, 62, 64) of a measuring device (10) for measuring a gas constituent of a gas present in a measuring chamber (56) of the measuring device (10), where the measuring chamber (56) is surrounded at least in part by the observation section (58, 60, 62, 64), the method comprising the following steps:
- Inlaying of a foil body (28, 30, 46, 50, 52, 54) with at least one foil layer (28, 30, 46, 50, 52, 54) in an injection-molding cavity,
- Injection of an observation injection-molded body (32, 34, 40) onto the foil body (28, 30, 46, 50, 52, 54) and thereby forming an observation wall component (58, 60, 62, 64), and
- Injection of a second injection-molded synthetic body (20) onto the observation wall component (22, 24, 26) and thereby fabricating the observation section (58, 60, 62, 64).

## Revendications

1. Dispositif de mesure (10) pour déterminer au moins un composant gazeux d'un gaz présent dans un espace de mesure (56) du dispositif de mesure (10), le dispositif de mesure (10) comprenant un boîtier (12) entourant l'espace de mesure (56), dont au moins une partie de paroi de boîtier est conçue comme partie d'observation (58, 60, 62) destinée à détecter le rayonnement électromagnétique émis par la partie d'observation (58, 60, 62, 64) en direction de l'extérieur de l'espace de mesure (56), la partie d'observation (58, 60, 62, 64) comprenant au moins une couche de film (28a, 28c, 46, 50, 52) et le boîtier (12) étant réalisé sous forme de boîtier moulé par injection en matière plastique,
**caractérisé en ce que** la partie d'observation (58, 60, 62, 64) comprend au moins un élément de paroi d'observation (22, 24, 26) comprenant un corps d'observation moulé par injection (32, 34, 36) moulé par injection sur au moins une couche de film (28a, 28c, 46, 50, 52) 46, 50, 52), le boîtier (12) comprenant l'au moins un élément de paroi d'observation (58, 60, 62) et un corps de cadre moulé par injection (20) moulé par injection sur l'élément de paroi d'observation (22, 24, 26).

2. Dispositif de mesure (10) selon la revendication 1,
**caractérisé en ce que** l'espace de mesure (56) peut être traversé par un flux de gaz le long d'une trajectoire virtuelle (SB).

3. Dispositif de mesure (10) selon la revendication 1 ou 2,
**caractérisé en ce que** le corps d'observation moulé par injection (32, 34, 40) présente un évidement d'observation (36, 38, 42) à l'intérieur duquel la au moins une couche de film (28a, 28c, 46, 50, 52) est accessible.

4. Dispositif de mesure (10) selon la revendication 3,
**caractérisé en ce qu'**une surface de bord (36a, 38a, 42a) de l'évidement d'observation (36, 38, 42) se rétrécit en direction de la au moins une couche de film (28a, 28c, 46, 50, 52) et est ainsi conçue comme surface de centrage pour un outil.

5. Dispositif de mesure (10) selon l'une des revendications précédentes, **caractérisé en ce que** la au moins une couche de film (28a, 28c, 46) est transparente au rayonnement électromagnétique dans le domaine spectral infrarouge.

6. Dispositif de mesure (10) selon la revendication 5,
**caractérisé en ce que** la au moins une couche de film (28a, 28c, 46, 50, 52) comprend un film polymère à orientation biaxiale (28c, 46).

7. Dispositif de mesure (10) selon l'une des revendications précédentes, **caractérisé en ce qu'**il comprend un premier et un deuxième composant de paroi d'observation au premier composant de gaz (22, 24) qui sont prévus sur le boîtier (12) pour détecter un premier composant de gaz de telle sorte qu'au moins une partie de l'espace de mesure (56) se trouve entre eux, le premier et le deuxième composant de paroi d'observation au premier composant de gaz (22, 24) sont disposés sur le boîtier (12) de telle sorte que le rayonnement électromagnétique qui est irradié à travers une partie d'observation (58 ou 60) de l'un des deux composants de paroi d'observation au premier composant de gaz (22, 24) dans l'espace de mesure (56) peut rayonner à travers la partie d'observation (60 ou 58) de l'autre élément de paroi d'observation au premier composant de gaz (22, 24) hors de l'espace de mesure (56).

8. Dispositif de mesure (10) selon l'une des revendications précédentes, **caractérisé en ce que** la au moins une couche de film (28a, 28c, 46, 50, 52) comprend, en tant que couche de film de mesure de température (52), un film métallique (52).

9. Dispositif de mesure (10) selon l'une des revendications précédentes, **caractérisé en ce que** la au moins une couche de film (28a, 28c, 46, 50, 52) en tant que couche de film de deuxième composant de gaz (50) pour détecter un deuxième composant de gaz différent du premier, présente une couche photoluminescente (50) avec au moins un luminophore intégré.

10. Dispositif de mesure (10) selon les revendications 8 et 9,
**caractérisé en ce qu'**il comporte un élément de paroi d'observation au deuxième composant de gaz (26) avec la couche de film pour le deuxième composant de gaz (50), qui comporte également la couche de film de mesure de la température (52).

11. Dispositif de mesure (10) selon l'une des revendications 9 ou 10,
**caractérisé en ce que** la couche de film pour le deuxième composant de gaz (50) est entièrement recouverte, sur son côté opposé à l'espace de mesure (56), par le corps d'observation moulé par injection (40), le corps d'observation moulé par injection (40) étant transparent au rayonnement électromagnétique dans la gamme de longueurs d'onde du rayonnement photoluminescent émis par la couche photoluminescente (50).

12. Dispositif de mesure (10) selon l'une des revendications précédentes, en incluant les revendications 7 et 9,
**caractérisé en ce qu'**il comprend à la fois le premier et le deuxième composant de paroi d'observation au premier composant de gaz (22, 24) qu'un deuxième élément de paroi d'observation au deuxième composant gazeux (26) avec la couche de film pour le deuxième composant gazeux (50), le premier et le deuxième élément de paroi d'observation au premier composant de gaz (22, 24) étant disposés sur le boîtier (10) (22, 24) chacun d'un côté différent de l'élément de paroi d'observation au deuxième composant gazeux (26).

13. Dispositif de mesure (10) selon l'une des revendications précédentes, **caractérisé en ce qu'**une formation de raccordement (16, 18) est disposée à au moins une extrémité longitudinale du boîtier, laquelle est conçue pour le raccordement d'un tuyau flexible et/ou d'une conduite.

14. Procédé de fabrication d'une section d'observation (58, 60, 62, 64) d'un dispositif de mesure (10) pour déterminer un composant gazeux d'un gaz présent dans un espace de mesure (56) du dispositif de mesure (10), l'espace de mesure (56) étant au moins partiellement entouré par la section d'observation (58, 60, 62, 64), le procédé comprenant les étapes suivantes :
- insertion d'un corps en feuille (28, 30, 46, 50, 52) avec au moins une couche de feuille (28a, 28c, 46, 50, 52) dans une cavité de moulage par injection,
- injection d'un corps d'observation moulé par injection (32, 34, 40) sur le corps en feuille (28, 30, 46, 50, 52, 54) et formation ainsi d'un composant de paroi d'observation (22, 24, 26), et
- moulage par injection d'un deuxième corps en plastique moulé par injection (20) sur l'élément de paroi d'observation (22, 24, 26) et réalisation ainsi de la section d'observation (58, 60, 62, 64).
